(19) Europäisches Patentamt
European Patent Office
Office européen des brevets

(11) **EP 1 804 670 B1**

(12) **EUROPEAN PATENT SPECIFICATION**

(45) Date of publication and mention of the grant of the patent:
**06.02.2013 Bulletin 2013/06**

(51) Int Cl.:
*A61B 8/00* (2006.01)    *G01S 15/89* (2006.01)
*G01S 7/52* (2006.01)    *A61N 7/00* (2006.01)

(21) Application number: **05771952.8**

(22) Date of filing: **15.08.2005**

(86) International application number:
**PCT/IL2005/000882**

(87) International publication number:
**WO 2006/018837 (23.02.2006 Gazette 2006/08)**

(54) **ULTRASONIC IMAGE-GUIDED TISSUE-DAMAGING**

ULTRASCHALLBILDGEFÜHRTE GEWEBESCHÄDIGUNG

ENDOMMAGEMENT DE TISSUS A GUIDAGE PAR IMAGE ULTRASONIQUE

(84) Designated Contracting States:
**AT BE BG CH CY CZ DE DK EE ES FI FR GB GR HU IE IS IT LI LT LU LV MC NL PL PT RO SE SI SK TR**

(30) Priority: **17.08.2004 US 601965 P**

(43) Date of publication of application:
**11.07.2007 Bulletin 2007/28**

(73) Proprietor: **Technion Research & Development Foundation Limited**
**32000 Haifa (IL)**

(72) Inventors:
• **AZHARI, Haim**
  **20142 Doar-Na Misgav (IL)**
• **AGNON, Yehuda**
  **20183 Doar-Na Misgav (IL)**
• **LEVY, Yoav**
  **37867 Yishuv Heinanit (IL)**

(74) Representative: **Dennemeyer & Associates S.A.**
**55, rue des Bruyères**
**1274 Howald (LU)**

(56) References cited:
WO-A2-99/40847      WO-A2-02/085178
US-A1- 2002 095 087    US-A1- 2003 040 698
US-A1- 2004 030 227

• **AZHARI H ET AL: "Volumetric imaging with ultrasonic spiral CT." RADIOLOGY JUL 1999 LNKD- PUBMED:10405752, vol. 212, no. 1, July 1999 (1999-07), pages 270-275, XP002594083 ISSN: 0033-8419**

**Description**

FIELD AND BACKGROUND OF THE INVENTION

The present invention relates to a system for damaging a target tissue and imaging a region containing the target tissue during a noninvasive, minimally invasive and/or invasive procedure.

Increased public awareness to breast cancer has led to widespread screening by mammography and to the early detection of cancer in many patients. Often, the tumors, which are detected, are relatively small, between 1 and 2 cm in size. Lumpectomy, the excision of the breast tumor with a limited amount of associated tissue, typically in combination with radiation therapy and chemotherapy, is the current mainstay of treatment. However, the procedure is invasive so as to scar the breast, and is thus cosmetically undesirable. Noninvasive surgical methods, free of surgical resection, are far more desirable.

Ablation, for example, by high intensity focused ultrasound (HIFU), microwaves or radiofrequency waves, offers an alternative to conventional lumpectomy [Randal J. High Intensity Focused Ultrasound Makes Its Debut. Journal of the National Cancer Institute. 2002 July 3, 94(13): 962-864; Hynynen K, Pomeroy O, Smith D, Huber P, McDannold N, Kettenbach J, Baum J, Singer S, Jolesz F. MR Imaging-Guided Focused Ultrasound Surgery Of Fibroadenomas In The Breast: A Feasibility Study, Radiology 2001 April,219:176-185; Gianfelice D, Khiat A, Amara M, Belblidia A, Boulanger Y. MR Imaging-Guided Focused Ultrasound Ablation Of Breast Cancer: Histopathologic Assessment Of Efficacy - Initial Experience. Radiology 2003, 227(3):849-855; Huber PE, Jenne JW, Rastert R, Simiantonakis I, Sinn HP, et al. A New Noninvasive Approach In Breast Cancer Therapy Using Magnetic Resonance Imaging-Guided Focused Ultrasound Surgery. Cancer Res 2001 Dec 1,61(23):8441-7; Gianfelice D, Abdesslem K, Boulanger Y, Amara M, Belblidia A, MR Imaging-Guided Focused Ultrasound Surgery (Mrigfus) Of Breast Cancer: Correlation Between Dynamic Contrast-Enhanced MRI And Histopathologic Findings. RSNA 2002; Tempany CMC, Stewart EA, McDannold N, Quade B, Jolesz F, Hynynen K. MRI Guided Focused Ultrasound Surgery (FUS) Of Uterine Leiomyomas: A Feasibility Study. Radiology 2003, 227:897-905; Wu F, Chen WZ, Bai J, Zou JZ, Wang ZL, Zhu H, Wang ZB. Tumor Vessel Destruction Resulting From High-Intensity Focused Ultrasound In Patients With Solid Malignancies. Ultrasound Med Biol 2002 Apr,28(4): 535-542; Madersbacher S, Schatzl G, Djavan B, Stulnig T, Marberger M. Long-Term Outcome Of Transrectal High-Intensity Focused Ultrasound Therapy For Benign Prostatic Hyperplasia. Eur. Urology. 2000, 37:687-694; and Non-Intrusive Measurement of Microwave and Ultrasound-Induced Hyperthermia by Acoustic Temperature Tomography, S. A. Johnson, D. A. Christansen, C. C. Johnson, J. F. Greenleaf and B. Rajagopalan, 1977, Ultrasonics Symposium Proceedings ,pp. 977-982].

When using high intensity focused ultrasound (HIFU), a special ultrasonic transducer or an array of transducers, located near the organ to be treated is used as a surgical "gun" to destroy in a noninvasive manner a small volume of tissue within the breast. The ultrasonic transducer is designed to focus a high-energy acoustic beam on the lesion, increasing the local temperature at the focal point to a temperature high enough to cause irreversible damage to the treated tissue. It is recognized that a HIFU procedure should be accompanied with an accurate imaging procedure so as to allow the physician or operator both to identify and to focus the focal point of the HIFU device on the tumor to be treated. It is also desirable to combine the HIFU with thermal mapping so as to monitor the ablation process.

Many imaging techniques are known in the art.

An MRI scanner, for example, can provide both an anatomical image and a thermal map of the treated organ [Hynynen K, Pomeroy O, Smith D, Huber P, McDannold N, Kettenbach J, Baum J, Singer S, Jolesz F. MR Imaging-Guided Focused Ultrasound Surgery Of Fibroadenomas In The Breast: A Feasibility Study, Radiology 2001 April,219: 176-185; Gianfelice D, Khiat A, Amara M, Belblidia A, Boulanger Y. MR Imaging-Guided Focused Ultrasound Ablation Of Breast Cancer: Histopathologic Assessment Of Efficacy - Initial Experience. Radiology 2003, 227(3):849-855; Huber PE, Jenne JW, Rastert R, Simiantonakis I, Sinn HP, et al. A New Noninvasive Approach In Breast Cancer Therapy Using Magnetic Resonance Imaging-Guided Focused Ultrasound Surgery. Cancer Res 2001 Dec 1,61(23):8441-7; Gianfelice D, Abdesslem K, Boulanger Y, Amara M, Belblidia A, MR Imaging-Guided Focused Ultrasound Surgery (Mrigfus) Of Breast Cancer: Correlation Between Dynamic Contrast-Enhanced MRI And Histopathologic Findings. RSNA 2002; and Tempany CMC, Stewart EA, McDannold N, Quade B, Jolesz F, Hynynen K. MRI Guided Focused Ultrasound Surgery (FUS) Of Uterine Leiomyomas: A Feasibility Study. Radiology 2003, 227:897-905].

However, most of these systems are expensive, and even in those medical institutes which do posses such scanners, considerations are oftentimes made regarding the expenses associated with the operation of the MRI scanners. Additionally, the access to the patient within the MRI scanner is limited. Another limitation of the use of MRI during HIFU is that the MRI scanner is very sensitive to ambient radiofrequency signals and its high magnetic field imposes severe limitations on the use of additional medical equipment, when needed.

Ultrasound offers a cost effective alternative imaging modality. The most common ultrasonic imaging technique is the pulse-echo ultrasound technique, also known as B-Scan. While this technique offers fair images of the anatomy it suffers from several inherent limitations, including inaccurate spatial mapping and poor signal-to-noise ratio (SNR).

U.S. Patent No. 6,500,121, for example, discloses a pulse-echo based acoustic transducer assembly which includes a single transducer and an imaging subsystem, a therapy subsystem and a temperature monitoring subsystem. The imaging subsystem generates an image of the treatment region, the therapy subsystem generates HIFU to ablate the treatment region, and the temperature monitoring subsystem maps and monitors the temperature of the treatment region. However, although the use of a single transducer is appealing from the standpoint of compactness, such configuration imposes a common unidirectional operation for both imaging and therapy subsystems. Since optimal imaging direction and optimal treatment directions do not necessarily coincide, single-transducer based systems must compromise on the imaging quality and/or ablation effectiveness.

U.S. Patent Application No. 20040030227 discloses a HIFU procedure to ablate a medical pathology in which the medical pathology is localized by acquiring at least two data sets of acoustic radiation, before and after a heating or cooling procedure, and comparing the data received from the two sets of scattered acoustic radiation. The pathology is detected from the temperature related changes.

Another imaging technique is based on transmission ultrasound (see, *e.g.*, U.S. patent No. 4,509,368), whereby the region-of-interest is imaged according to its transmission characteristics (as opposed to the reflective characteristics of the B-scan). Transmission ultrasound has a significantly higher SNR as compared to the B-scan technique.

Also known is a technique known as inverse scattering (see, *e.g.*, U.S. Patent Nos. 6,636,584, 6,587,540, 6,005,916 and 5,588,032), in which information of the region of interest is obtained by allowing wavefields to interact with the region-of interest and analyzing the scattering trajectories of the wavefields.

An additional imaging technique is thermoacoustic computed tomography (see, *e.g.*, U.S. Patent No. 6,216,025) which is an hybrid imaging technique that converts incident electromagnetic energy into sound waves that can be used to reconstruct the absorption pattern of the incident energy source.

Also of prior art of interest are U.S. Patent Application No. 2005/0038339 and U.S. Patent Nos. 6,716,184, 6,685,639, 6,280,402, 6,216,025, 5,769,790, 5,558,092 and 4,932,414.

WO 99/40847, which is considered to form the closest prior art document, discloses an ultrasound imaging apparatus comprising a first source of ultrasound energy at a first frequency which irradiates a region of a body, a second source of ultrasound energy at a second frequency which irradiates said region of the body, wherein the energy at the first and second frequencies at the region are sufficient to cause energy at at least one of a third frequency and a fourth frequency to be generated in the region, said third frequency being the difference between the first and second frequencies and the fourth frequency being the sum of the first and second frequencies, and a directional antenna which receives energy at one of the third and fourth frequencies from the region and provides an image signal in response thereto.

The present invention provides solutions to the problems associated with prior art HIFU techniques.


SUMMARY OF THE INVENTION

It is the object of the present invention to provide a system which provide an accurate two- or thee-dimensional anatomical image of a region containing a target tissue to be treated, *e.g.*, by ablation or cavitation.

It is further the object of the present invention to provide a system which allow the physician or operator to identify the target tissue.

It is further the object of the present invention to provide a system which allow the physician or operator to mark the spatial coordinates of the target tissue.

It is further the object of the present invention to provide a system which allow the operator to locate the high intensity focused ultrasound (HIFU) focal region, before activating the HIFU system at high intensity. As the speed of sound, attenuation and other acoustic properties depend on the type of tissue, such focusing needs be done for each case, and for each tissue type specifically.

It is further the object of the present invention to provide a system which allow the operator to focus the HIFU so that peak ablation temperatures are on the target tissue, so as to efficiently destroy the target tissue once the HIFU is activated at full power.

It is further the object of the present invention to provide a system for obtaining a temperature image (thermal map) during the treatment procedure so as to monitor the damaging process.

It is further the object of the present invention to provide a system for obtaining an image of the region after the procedure, for verification, to ensure the success of the treatment.

According to an additional aspect of the present invention there is provided a HIFU system according to claim 1.

According to yet an additional aspect of the present invention there is provided a system for damaging a target tissue according to claim 2.

According to further features in preferred embodiments of the invention described below, the imaging system comprises a TUCT system for imaging the region by TUCT.

According to still further features in the described preferred embodiments the imaging system and the HIFU device are designed and constructed to operate substantially contemporaneously.

According to further features in preferred embodiments of the invention described below, the TUCT system comprises an intracorporeal ultrasound device, an extracorporeal ultrasound device, and a data processor for analyzing ultrasonic radiation transmitted between the intracorporeal ultrasound device and the extracorporeal ultrasound device so as to generate an image of the region.

According to still further features in the described preferred embodiments the damaging comprises ablation.

According to still further features in the described preferred embodiments the damaging comprises cavitation.

According to still further features in the described preferred embodiments the intracorporeal ultrasound device is adapted to be inserted through the anus. According to still further features in the described preferred embodiments the intracorporeal ultrasound device is adapted to be inserted through the vagina. According to still further features in the described preferred embodiments the intracorporeal ultrasound device is adapted to be inserted through the urethra. According to still further features in the described preferred embodiments the intracorporeal ultrasound device is adapted to be inserted through the esophagus.

According to still further features in the described preferred embodiments the intracorporeal ultrasound device is mounted on a transport mechanism. According to still further features in the described preferred embodiments the transport mechanism is selected from the group consisting of an endoscopic probe and a catheter.

According to still further features in the described preferred embodiments the imaging system is operable to employ pulse-echo imaging. According to still further features in the described preferred embodiments the imaging system is operable to employ inverse scattering imaging. According to still further features in the described preferred embodiments the imaging system is operable to employ magnetic resonance imaging. According to still further features in the described preferred embodiments the imaging system is operable to employ thermoacoustic computerized tomography.

According to still further features in the described preferred embodiments the TUCT comprises analysis of frequency harmonics. According to still further features in the described preferred embodiments the TUCT comprises analysis of frequency combinations. According to still further features in the described preferred embodiments the TUCT comprises analysis of frequency harmonic combinations. According to still further features in the described preferred embodiments the TUCT is obtained by spiral scanning. According to still further features in the described preferred embodiments the TUCT comprises analysis of time-of-flight. According to still further features in the described preferred embodiments comprises analysis of phase shift. According to still further features in the described preferred embodiments the TUCT comprises analysis of frequency-dependent velocity dispersion.

According to still further features in the described preferred embodiments the target tissue forms at least a part of a tumor or a part of a malignant tumor.

According to still further features in the described preferred embodiments the target tissue is a pathological tissue.

According to still further features in the described preferred embodiments the target tissue is a part of a breast, a thigh, a fatty tissue, a testicle, the prostate, the bladder, a lower abdomen organ, mid abdomen organ, the tongue, the brain, the liver, a kidney, the stomach, the pancreas, the esophagus, the uterus or the ovary.

The present invention successfully addresses the shortcomings of the presently known configurations by providing method and system for imaging a region and damaging a target tissue present in the region.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

Implementation of the system of the present invention involves performing or completing selected tasks or steps manually, automatically, or a combination thereof. Moreover, according to actual instrumentation and equipment of preferred embodiments of the system of the present invention, several selected steps could be implemented by hardware or by software on any operating system of any firmware or a combination thereof. For example, as hardware, selected steps of the invention could be implemented as a chip or a circuit. As software, selected steps of the invention could be implemented as a plurality of software instructions being executed by a computer using any suitable operating system. In any case, selected steps of the system of the invention could be described as being performed by a data processor, such as a computing platform for executing a plurality of instructions.

BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

FIG. 1 is a flowchart diagram illustrating a method for determining a focal region of high intensity focused ultrasound (HIFU);

FIG. 2 is a flowchart diagram illustrating a method for damaging a target tissue of a subject;

FIG. 3 is a schematic illustration of a relevant geometry for a transmission-ultrasound computerized tomography (TUCT) system, according to a preferred embodiment of the present invention;

FIG. 4 schematically illustrates a relevant geometry for a TUCT system, in which the region is a breast, according to a preferred embodiment of the present invention;

FIG. 5 is a flowchart diagram of a method for imaging a region of a subject;

FIG. 6A is a schematic illustration of intracorporeal and extracorporeal ultrasound devices used for imaging the stomach by TUCT, according to a preferred embodiment of the present invention;

FIG. 6B is a schematic illustration of intracorporeal and extracorporeal ultrasound devices used for imaging the prostate or bladder by TUCT, according to a preferred embodiment of the present invention;

FIG. 6C is a schematic illustration of intracorporeal and extracorporeal ultrasound devices used for imaging the uterus, bladder or ovary by TUCT, according to a preferred embodiment of the present invention;

FIG. 7 is a flowchart diagram of a method for TUCT guided damaging of a target tissue;

FIG. 8 illustrates a three-dimensional breast phantom reconstruction based on attenuation-coefficient imaging, as obtained by the TUCT system of Figure 4;

FIGs. 9A-C schematically illustrate configurations for producing an anatomical and temperature image (thermal map) using a TUCT system, according to a preferred embodiment of the present invention;

FIGs. 10A-C schematically illustrate the configuration of Figure 9A, designed for imaging and guiding a HIFU thermal procedure in a woman's breast, according to a preferred embodiment of the present invention;

FIG. 11 illustrates a temperature image (thermal map) as obtained by TUCT system 32, according to a preferred embodiment of the present invention;

FIGs. 12A-C depict an ablated region and its corresponding dispersion and time of flight images for an in-vitro tissue specimen obtained by a TUCT system, according to a preferred embodiment of the present invention;

FIGs. 13A-B depict a lateral view of an actual in-vivo scan of a breast with papiloma growth, as obtained by a TUCT system, using third harmonic band imaging (Figure 13A) and first harmonic band imaging (Figure 13B), according to a preferred embodiment of the present invention;

FIG. 14 schematically illustrates a configuration for a TUCT system, according to a preferred embodiment of the present invention; and

FIG. 15 depicts a focal point in a target, identified by transmitting two frequencies and receiving a frequency which equals the sum of the two frequencies, according to a preferred embodiment of the present invention.

## DESCRIPTION OF THE PREFERRED EMBODIMENTS

The present invention is a system which can be used in image-guided procedures for damaging a target tissue. Specifically, the present invention can be used for damaging the target tissue by ablation or cavitation and imaging a region containing the target tissue during a noninvasive, minimally invasive and/or invasive procedure.

The principles and operation of the present invention may be better understood with reference to the drawings and accompanying descriptions.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein are for the purpose of description and should not be regarded as limiting.

The propagation of waves, such as ultrasonic waves, through a medium is a complex phenomenon which is typically described mathematically by means of one or more wave equations relating the characteristics of the medium with the characteristics of a perturbation formed therein. These equation express the action of restoring forces on molecules of the medium which are displaced from their equilibrium. In most fluidic media, when the perturbation is sufficiently small, say, smaller or of the order of the bulk module of the fluid, the restoring forces are proportional to the displacements and the governing wave equation is a linear partial differential equation. In these cases it is said that the medium or the wave phenomenon is linear. In these media, the principle of linear superposition, according to which several different waves can propagate independently through the same medium, is maintained. In linear medium the perturbation extent (*e.g.*, pressure, displacements) at any given time and place is the linear sum of the displacements due to each of the simultaneously propagating waves.

When a perturbation formed in the fluid is not small, the restoring forces present in the medium depend on higher powers of the displacement of the molecules from equilibrium. In such cases, the wave equations are not linear partial differential equations, and it is said that the medium or wave phenomenon is nonlinear. In nonlinear medium, the principle of linear superposition breaks, and there is no independent propagation of waves within the medium. When the medium becomes nonlinear, the interaction of two or more waves results in generation of several harmonics and harmonic combinations which were not present in the original waves before the interaction.

While conceiving the present invention it has been hypothesized and while reducing the present invention to practice it has been realized that nonlinear wave phenomena can be exploited for determining a focal region of high intensity focused ultrasound (HIFU). Such determination is particularly useful when HIFU is used in for ablation or cavitation of a target tissue. The determination of the focal region can allow the HIFU operator to locate the focal point or hot-spot before the activation of the damaging process, thereby to ensure that when the HIFU device operates at high intensity, the peak temperature or the cavitation region is focused on or close to the target tissue.

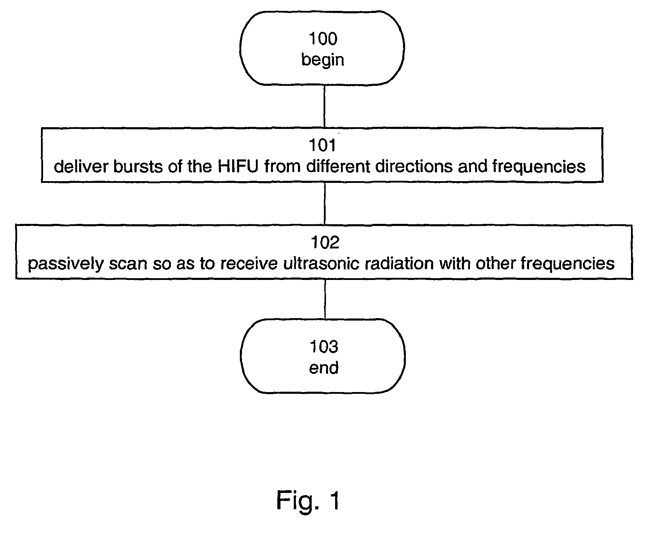

It is to be understood that, unless otherwise defined, the method steps described hereinbelow can be executed either contemporaneously or sequentially in many combinations or orders of execution. Specifically, the ordering of the flowcharts of Figures 1, 2, 5 and 7 below, is not to be considered as limiting. For example, two or more method steps, appearing in the following description or in a particular flowchart in a particular order, can be executed in a different order (*e.g.*, a reverse order) or substantially contemporaneously.

Referring now to the drawings, Figure 1 is a flowchart diagram illustrating a method for determining a focal region of HIFU, according to a preferred embodiment. The method begins at step **100** and continues to step **101** in which bursts of the HIFU are delivered from a plurality of directions and at a plurality of different frequencies to a region, for example, a region containing the target tissue. The duration and intensity of the bursts are preferably selected below the ablation threshold but above the nonlinear threshold. In other words, the bursts should be of a sufficiently high intensity so as to onset nonlinear wave phenomena, substantially without causing damage to the tissue. This can be done, for example, by operating the HIFU device at a fraction of its power and/or for short duration. Typical values for the intensity of the HIFU bursts at the focal point are from a few Watts/cm$^2$ to several hundreds of Watts/cm$^2$.

The duration of the HIFU bursts depends on the applied intensity. Specifically, for higher intensities, the preferred duration is shorter. Typical values for the duration is from a few microseconds seconds to a several seconds.

Since the bursts, as stated, onset nonlinear wave phenomena, harmonics and harmonic combinations are generated. Theoretically and experimentally, it has been uncovered that the acoustic energy carried by the harmonics is maximal at the focal region the HIFU device, provided there are no strong reflectors in the vicinity. Each harmonic is characterized by a frequency other than the frequencies of the original bursts. The method continues to step **102** in which the region is passively scanned so as to receive ultrasonic radiation having at least one frequency other than the original frequency, and the focal region of the HIFU is thus determined. According to a preferred embodiment of the present invention, the harmonic frequencies are combinations (*e.g.*, linear combinations) of the original frequencies. For example, when two frequencies $f_1$ and $f_2$ are employed, the harmonic combination $f_h$ can be at a frequency of $f_h = m\,f_1 + n\,f_2$, where $m$ and $n$ are arbitrary integer coefficients.

The focal region can then be determined by employing conventional parallax or triangulation methods on the directions from which the harmonic or harmonic combination is received. As there is more than one harmonic or harmonic combination, lower frequencies are preferred so as to minimize attenuation hence improve the accuracy. For two frequencies, the preferred frequency for determining the focal region is $f_1 - f_2$, corresponding to $m = 1$ and $n = -1$ in the above definition of $f_h$.

In various exemplary embodiments of the invention $f_1$ and $f_2$ may be from 10 % apart to 100 % apart. Other values and ranges are also contemplated. Thus, for example, $f_2 = 1$ MHz and $f_2 = 1.1$ MHz, or $f_1 = 1.5$ MHz and $f_2 = 3$ MHz.

The determination of the HIFU focal region can be combined in any invasive, minimally invasive or noninvasive damaging procedure, including, without limitation, the damaging procedures further detailed hereinunder.

The method ends at step **103.**

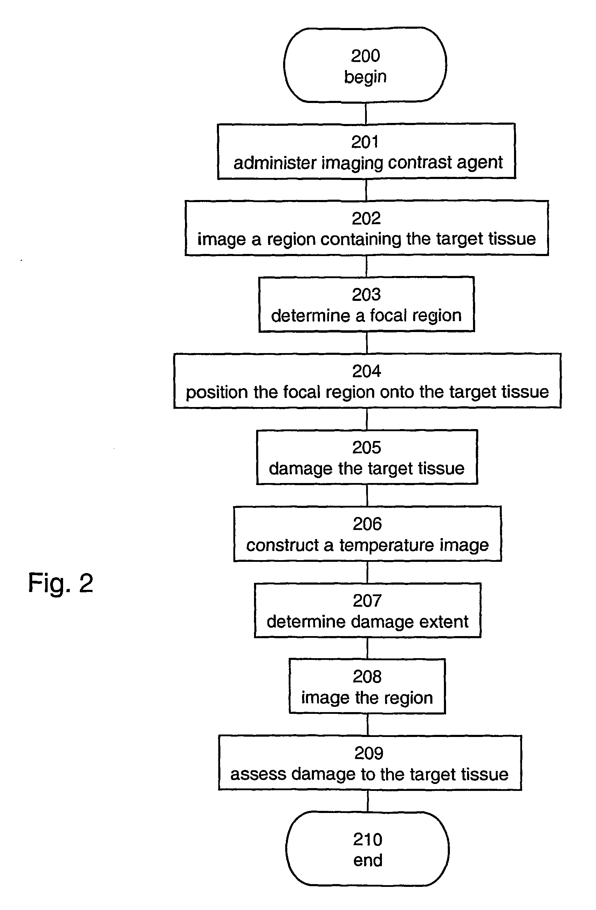

Reference is now made to Figure 2 which is a flowchart diagram illustrating a method for damaging a target tissue of a subject, according to a preferred embodiment. The method begins at step **200** and, optionally and preferably continues to step **201** in which an effective amount of imaging contrast agent is administered to the subject. The administration can be via any transport mechanism, such as, but not limited to, a catheter or a needle. The type of imaging contrast agent depends on the imaging technique.

For example, when MRI is employed, the contrast agent is an MRI contrast agent, which can be either a positive or a negative MRI contract agent, where "positive" contract agent increases the signal while "negative" contract agent decreases the signal relative to nearby tissues or fluids. Positive MRI contrast agents are typically used such that their dominant effect is to reduce the $T_1$ relaxation time, while negative MRI contrast agents typically are used such that their dominant effect is to reduce the $T_2$ relaxation time.

When the imaging is by acoustically based, the imaging contrast agent is an acoustical detectable fluid, typically in gaseous state, but can also be mixed in a liquid solution. Such substances are known in the art and found, *e.g.*, in U.S. patent Application No. 20030105402. Representative examples for imaging contrast agent suitable for ultrasound imaging include, without limitation, halogenated hydrocarbon, halogenated alkane gases, nitrogen, helium, argon, xenon and the like. Perfluorinated hydrocarbon represents a preferred halogenated alkane gas for its acoustic properties as well as its low toxicity. Perfluorinated hydrocarbon may be a saturated perfluorocarbon, an unsaturated perfluorocarbon and/or a cyclic perfluorocarbon.

The method continues to step **202** in which the region containing the target tissue is imaged. The region can be imaged to provide two- or three-dimensional image, using any conventional imaging system. Representative examples include, without limitation, acoustic-based imaging, inverse scattering imaging and magnetic resonance imaging (MRI). Also contemplated are various combinations between different imaging techniques. This embodiment is particularly (but not exclusively) useful when more than one imaging technique can be employed using the same imaging system. For example, in ultrasound imaging, the same system can be used both for pulse-echo ultrasound imaging and for transmission-ultrasound computerized tomography (TUCT). Thus, pulse-echo image can be followed by a TUCT image. The advantage of this embodiment is that the use of two or more imaging techniques increases the amount of information which can be obtained from the region. Additionally, in this embodiment, fast acquisition of an image with lower quality can precede the acquisition of more accurate image so as to quickly assess the location of the region of interest.

Subsequently to or contemporaneously with the imaging step, the method preferably continues to step **203** in which the focal region of the HIFU is determined as further detailed hereinabove in conjunction with the flowchart diagram of Figure 1. The method continues to step **204** in which the focal region us positioned onto the target tissue. This can be achieved by redirecting the HIFU device and repeating step **203** until the focal region overlaps, to a predetermined degree of accuracy, with the target tissue. This procedure is preferably executed contemporaneously with the imaging step (step **202**) so as to obtain a real-time imaging and focusing of the ultrasonic radiation on the target tissue.

Once the HIFU focal region is positioned on the target tissue, the method continues to step **205** in which the tissue is damaged by high intensity ultrasonic radiation. The ultrasonic radiation can have any power which is sufficient to damage the tissue, by ablation or by cavitation. When the tissue is damaged by ablation, the ultrasonic radiation increases the temperature of the target tissue to above the characteristic ablation temperature of the tissue, which is typically about 57 °C.

As used herein "about" refers to $\pm$ 20 %.

When the tissue is damaged by cavitation, the ultrasonic radiation form small cavities in liquids present in or neighboring the target tissue. The formed cavities instantaneously collapse and the process effects localized agitation which causes cavitation damage to the target tissue.

According to a preferred embodiment the method continues to step **206** in which a temperature image (thermal map) of the region is constructed. This can be done by employing any temperature imaging technique known in the art, including, without limitation, acoustic radiation, magnetic resonance, arrays of temperature sensors (see, *e.g.*, U.S. Patent No. 6,916,290), and the like. A detailed description of a preferred procedure for constructing a temperature image by transmission-ultrasound computerized tomography (TUCT), is provided hereinafter. The temperature image can be used for, for example, for damage control.

Thus, according to a preferred embodiment the method continues to step **207** in which the damage extent to the region is determined. In this embodiment, the temperature image is preferably constructed substantially contemporaneously or immediately after the damaging step (step **205**) so as to so as to control the damage to the region. When the damage extent satisfies a predetermined condition (*e.g.*, a maximal area which is allowed to be heated) the damaging step is ceased.

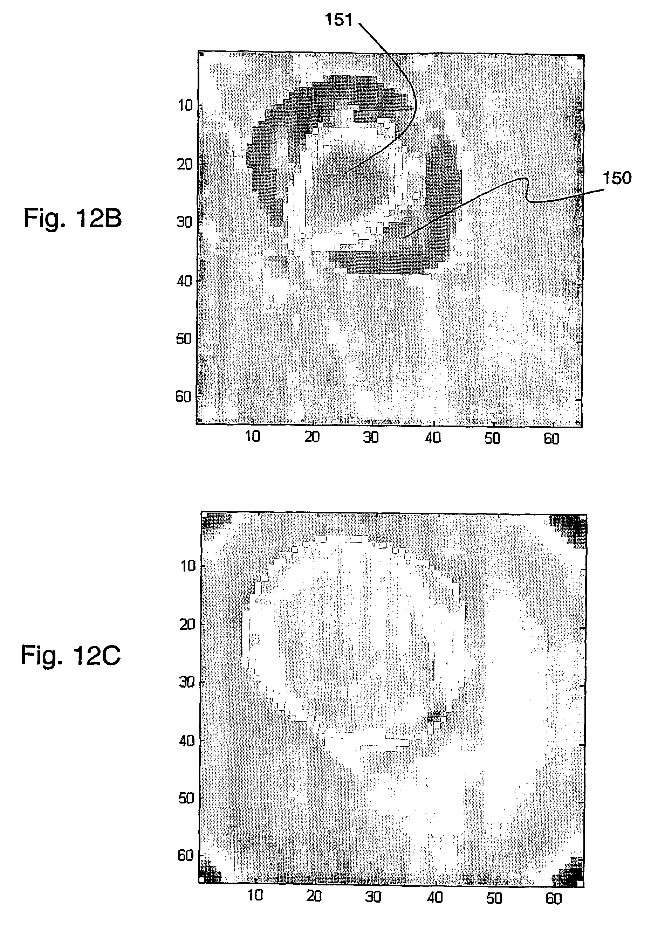

In various exemplary embodiments the method continues to step **208** in which the region is imaged subsequently to the damaging step. This embodiment is particularly useful for performing damage assessment. Thus, the method preferably continues to step **209** in which the damage to the target tissue and/or region is assessed based on the images. According to the presently preferred embodiment the image is constructed a few minutes after treatment to ensure the success of the treatment. It was found by the present inventors that efficient damage assessment can be obtained when the TUCT comprises analysis of velocity dispersion as further detailed hereinunder (see Equation 7 and Figure 12B in the Examples section that follows).

The method end at step **210.**

Of the imaging techniques delineated above, acoustic-based imaging is more preferred from the standpoints of cost, availability and safety. Many types of acoustic-based imaging are contemplated, including, without limitation, transmission-ultrasound computerized tomography (TUCT), pulse-echo ultrasound and thermoacoustic imaging.

Clinically available ultrasonic scanners are typically based on the pulse-echo technique, also known as B-Scan. Although this technique offers fair images of the anatomy, it is somewhat less preferred for the following reasons.

First, spatial mapping by the pulse-echo technique is based on the assumption that the speed of sound is the same for all types of tissues. This assumption introduces errors in geometrical measurements, since variations in the order of 5 %, in speed of sound between different tissue types (*e.g.*, breast fat and parenchyma) are common.

Second, the pulse-echo technique does not provide quantitative acoustic properties of the tissue, since the echoes detected by the system are affected not only by the tissue of interest but also by the unknown acoustic properties of all tissue regions along the path of the acoustic beam.

Third, the signal-to-noise ratio (SNR) is relatively poor since the reflection coefficient of soft tissues is small and the appearance of image noise, such as "speckle noise," is common.

Fourth, although the pulse-echo technique can, in principle, be used for generating temperature images (thermal map), the resulting because temperature images are of insufficient reliability, due to the low accuracy of the imaging process.

Another imaging technique which is contemplated is thermoacoustic imaging. Thermoacoustic imaging is known in the art and is found, *e.g.*, in U.S. Patent No. 4,385,634. Briefly, the technique utilizes acoustic wave which are generated by applying sudden thermal stress to the region. The generated acoustic wave carriers information on the composition and structure of the region.

The sudden thermal stress can be induced by a pulse of radiation which deposits energy causing a rapid, but very small, rise of temperature , typically, of order of a few to a few tens of micodegrees centigrade. The radiation may be ionizing radiation (*e.g.*, high energy electrons, photons, neutrons), or, more preferably non-ionizing radiation (*e.g.*, radiofrequency electromagnetic radiation, microwave electromagnetic radiation or ultrasonic radiation). Thermoacoustic imaging is particularly useful for soft-tissue regions because such regions, while having sufficient inhomogeneities to produce image features or structure, are sufficiently homogeneous to allow the thermoacoustic waves to reach the surface of the region with small attenuation due to scattering and absorption. The acoustic waves can be detected by one or more passive ultrasound transducers contacting or being in close proximity to the surface of the region, and the information collected by the transducers can be processed by CT techniques (*e.g.*, using Radon transform) to obtain an image of the region.

A more preferred imaging technique is TUCT, which is advantageous over the pulse-echo technique, basically, but not exclusively, because in TUCT no assumptions are made on tissue properties and the inaccuracy inherent to the pulse-echo technique is thus avoided. Furthermore, SNR via TUCT is superior to that obtained via the pulse-echo technique.

An additional advantage of TUCT is that the different physical properties of the transmitted waves, as detected by the receiving transducer, can yield various aspects of anatomical images and may further be used for imaging the damaged region. The topographic images obtained by TUCT can depict images of various acoustic properties of the tissue, such as attenuation coefficient, speed of sound, acoustic refractive index, phase shift and others.

TUCT is also superior to the pulse-echo technique with respect to thermal mapping. This is because there are many acoustical properties which depend on the temperature and to which TUCT is sensitive. One such property is the speed of sound, which can be measured, for example, by "time-of-flight" technique (see, *e.g.*, Rajagopalan, B.; Greenleaf, J. F.; Thomas, P. J.; Johnson, S. A.; Bahn, R. C.: Variation of acoustic speed with temperature in various excised human tissues studied by ultrasound computerized tomography, Ultrasonic Tissue Characterisation II , EDITOR- Linzer, M, PP. 227-33, 1979 ; Jossinet, J.; Cathignol, D.; Chapelon, J. Y.; Dittmar, A.; Schmitt, M.: Practical temperature measurements by ultrasound tomography. Journal de Biophysique & Medicine Nucleaire , VOL. 7, NO. 5, PP. 179-83, 1983 ; Mizutani, K., Nishizaki, K., Nagai, K., Harakawa, K., "Measurement of temperature distribution in space using ultrasound computerized tomography," Japanese Journal of Applied Physics, Part 1 VOL. 36, NO. 5B, May 1997, PP. 3176-7). Other acoustical properties which are contemplated include, without limitation, phase shift and frequency-dependent velocity dispersion.

The term "Computerized Tomography" (CT) refers to a method of producing a two or three-dimensional image of the internal structures of a solid object, such as a human body, by recording changes produced in waves, such as x-ray or ultrasound waves, when transmitted through the object. More specifically, it relates to an image of a body structure, constructed by a computer from a series of projections, as produced by the transmitted waves, along an axis.

Generally, in CT a mathematical reversible transform, such as Radon or an equivalent transform (*e.g.*, exponential projections which are used in SPECT), is used for the reconstruction of the two or three-dimensional image.

In essence, the Radon operator maps the spatial domain (*x*, *y*, *z* in Cartesian coordinates) to the projection domain ($\rho$, $\theta$ in polar coordinates), such that each point in the projection domain corresponds to a straight line integral in the spatial domain, and each point in the spatial domain becomes a sine curve (also known as a sinogram) in the projection domain.

Formally, the Radon transform of a function $f(x, y)$ is defmed the line integral of $f$, where the integration contour is a straight line $\rho = x \cos\theta + y \sin\theta$, defined by its distance, $\rho$, from the origin and its angle of inclination $\theta$:

$$r(\rho,\theta) = \iint f(x,y)\delta(x\cos\theta + y\sin\theta - \rho)dx\,dy \qquad (EQ.\ 1)$$

where δ is the Dirac delta function which defines integration over the line.

In CT, the inverse of the Radon transform is used to reconstruct images (commonly via a filtered back-projection algorithm) in two- or three-dimensions from intensities recorded in one or two dimensions respectively.

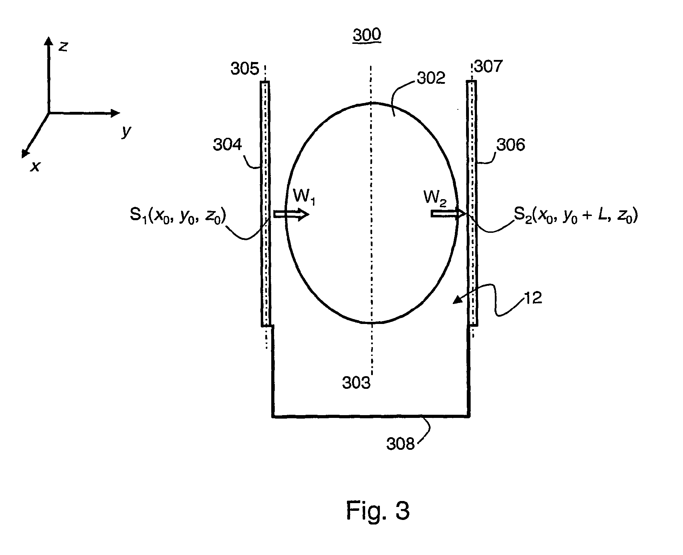

Figure 3, schematically illustrates a relevant geometry for a TUCT system **300,** in accordance with preferred embodiment of the present invention. A region **302** to be imaged is positioned between two or more ultrasound devices. Shown in Figure 3 is a planar view in the *y-z* plane of region **302** and two ultrasound devices, designated by numerals **304** and **306.** The z axis is typically defined by a longitudinal axis **303** of region **302.** For example, when region **302** is a breast, the *z* axis is parallel to the longitudinal axis of the breast. Any one of devices **304** and **306** can be either extracorporeal or intracorporeal, as further detailed hereinunder. Each ultrasound device can be provided as a single ultrasound transducer or an array of two or more ultrasound transducers. Preferably, but not obligatorily, region **302** is devoid of bones or air. Thus, region **302** can be a breast, tongue, thigh, fatty tissue, testicle, prostate, lower abdomen organ, mid abdomen organ or any part thereof.

Ultrasound devices **304** and **306** are preferably coupled to region **302** via a tissue coupling medium **12** which matches the characteristic impedances of the body of the ultrasound devices and the tissue of region **302.** Medium **12** can be, for example, water, ultrasound gel and the like.

Each of the ultrasound devices can be in a form of a single transducer, or an array of transducers, as desired. In the exemplified configuration of Figure 3, devices **304** and **306** are positioned on opposite sides of region **302.** Devices **304** and **306** oppose one another in a transmitter-receiver configuration, preferably by a 180° arrangement so as to minimize undesired scattering effects. This can be achieved, for example, by connecting the respective devices by a bridge **308.** Alternatively, a calibration procedure may be employed by transmitting calibration pulses and moving the devices to a position in which the readings from scattering radiation are minimized. When one of the devices is intracorporeal, the positioning of the device can also be done using an additional imaging technique, as further detailed hereinunder.

The ultrasound devices are arranged such that at any given time in which system **300** is operative, one is operative as a transmitter and the other as a receiver to an ultrasonic wave transmitted through the region. It will be appreciated that when desired, both devices may operate as receivers or as transmitters, and may rapidly interchange their roles during a data-acquisition procedure.

An ultrasonic wave $W_1(t,f)$, which varies in time $t$ and frequency $f$ and which may be a continuous wave (CW) or a pulse, is transmitted from one device, say device **304,** through region **302,** to be detected as an ultrasonic wave $W_2(t, f)$ by the other device (in the present example device **306**). As a result of the interaction of the transmitted ultrasonic wave $W_1$ with the tissues in organ **302,** the properties of the received wave $W_2$ differ from those of the transmitted wave $W_1$.

In general, a relationship between the detected wave $W_2(t, f)$ and the transmitted wave $W_1(t, f)$, through a specific tissue type, such as a healthy muscle tissue, a cancerous tissue, a bone tissue, an ablated tissue or the like, may be used as a "tissue signature" of that specific tissue type, since it is unique for each tissue type. In this manner, an anatomical structure may be obtained based on the tissue signature of the transmitted wave.

Any acoustic property g of the wave transmitted from point $S_1(x_0, y_0, z_0)$, and received at point $S_1(x_0, y_0 + L, z_0)$, where $L$ is the distance between the transmitting and receiving devices, can generally have a spatial dependence and/or a frequency dependence containing information regarding region **302.** Given such acoustic property, a mathematical operator $F(W_1, W_2)$ can be applied to provide a point projection $p(x_0, y_0)$ of $g = g(x, y, z; f)$ as follows:

$$p(x_0, z_0) = F(W1, W2) = \int_{y=y_0}^{y=y_0+L} g(x_0, y, z_0, f) \cdot dy \qquad \text{(EQ. 2)}$$

where, in a more general case, frequency $f$ can be replaced by one or more frequency bands. Preferably, the acoustic property $g$ is at least locally integrable in the domain from $S_1$ and $S_2$, more preferably $g$ is integrable in the domain from $S_1$ and $S_2$.

Many acoustic properties and associated point projections are contemplated, and further detailed hereinunder.

According to a preferred embodiment of the present invention, for a given height $z_0$, region **302** is scanned at a plurality of points along a straight line, say along the x axis, to thereby obtain a line projection $p(x, z_0)$ [not to be confused with point projection $p(x_0, z_0)$] of the region's property $g$ at $z_0$. The sampling density along the straight line is preferably at least one point per millimeter, more preferably at least 5 points per millimeter.

As the line projections are collected by rotating the frame of reference consecutively by an incremental angle $\Delta\theta$, one obtains a Radon transform of region at the height $z_0$. Then, by calculating the inverse Radon transform (*e.g.* , by filtered back projection or algebraic reconstruction methods) the computed topographic cross section of the region at the height $z_0$, can be obtained.

In order to obtain a three-dimensional image in a conventional CT scan, the z coordinate is incremented between acquisitions, such that a series of computed topographic cross sections of the object at the heights $z_0, z_1, z_2$ ... are be

obtained. Preferably, but not obligatorily, different heights are equally spaced, *i.e.*, $z_{i+1}= z_i + \Delta z$, where $i$ is a non-negative integer and $\Delta z$ is a predetermined increment parameter. The three-dimensional image of $g(x, y, z; f)$ for the region can then be reconstructed by stacking the images one atop the other.

Alternatively, the z coordinate can be varied substantially continuously (by decreasing or nulling the vertical distances between successive heights in the above series) thereby to allow implementation of spiral CT algorithms for reconstructing a three-dimensional image of the acoustic property, g. The main advantages of continuous variation of z are that data acquisition for obtaining a three-dimensional image is faster and that small lesions and (or) targets located between increments, which can be missed by conventional CT, are more likely to be detected by the continuous coverage. Spiral transmission ultrasound computerized tomography (SUCT) has been recently offered as a new volumetric imaging method for the breast [Azhari H, Sazbon D: Volumetric imaging using spiral ultrasonic computed tomography. Radiology, 1999, 212(1):270-275.] With SUCT, quantitative three-dimensional reconstructions of a region are obtained in a manner similar to spiral X-rays CT, without the hazardous X-ray ionizing radiation.

The acquisition of a series of images along the z axis can be utilized in more than one way. In one embodiment, by positioning the ultrasound devices perform a linear motion along the z axis, and transmit and/or receive the ultrasound waves while moving (in case of continuous variation of the z coordinate) or at a plurality of different positions (in case of discrete variation of the z coordinate). In another embodiment, the ultrasound devices are elongated and capable of simultaneous acquisition of information along their longitudinal axis. In this embodiment, the devices are preferably positioned such that their longitudinal axes (designated **305** and **307** in the exemplified configuration of Figure 3) are parallel to the z axis, which, as stated, is typically defined by longitudinal axis **303** of region **302.** In still another embodiment, the elongated ultrasound devices comprise an array of ultrasound transducers positioned along the longitudinal axes of the ultrasound devices, such that using phased array beam-forming techniques different images at different z coordinates can be rapidly obtained. The individual ultrasound transducers can operate either independently or they can be synchronized to transmit/receive the ultrasound radiation at a predetermined time ordering. Alternatively, the individual transducers can operate simultaneously for fast acquisition.

In accordance with the present embodiments, topographic images of tissue temperature may similarly be obtained, as tissue temperature may be correlated with several acoustic properties. More particularly, when one desires to investigate a change in an acoustic property, induced by a temperature-related surgical procedure, for example, ultrasound or microwave ablation, one may obtain a set of reference images of the acoustic property at $t = t_0$, denoted as IM[$g(x, y, z; f\ t = t_0)$], prior to the surgical procedure, and a set of images of the acoustic property, during or after the tissue is heated at $t = t_1$, denoted IM[$g(x, y, z;$ f; $t = t_0)$]. The temperature-induced change $\Delta g$ in the acoustic property can be mapped by subtracting the two sets:

$$\Delta g(x, y, z) = \mathrm{IM}[g(x, y, z; f; t = t_1)] - \mathrm{IM}[g(x, y, z, f; t = t_0)] \quad (\mathrm{EQ.}\ 3)$$

Then, using an experimentally defined transformation operator $U$, a local temperature $T(x, y, z)$ can be related to the corresponding change in the acoustic property:

$$T(x, y, z) = U[\Delta g(x, y, z)]. \quad\quad (\mathrm{EQ.}\ 4)$$

Acoustic properties with which temperature may be correlated, for example, in order to monitor ultrasound ablation, include time-of-flight, phase shift, frequency-dependent velocity dispersion, that is, changes in the speed of sound as a function of frequency, and high-harmonic band energies, as further detailed hereinunder.

As stated, the ultrasound devices employed during the TUCT oppose each other in a transmitter receiver configuration. The TUCT images of the present embodiments can be obtained for organs provided the interspace between the ultrasound devices is devoid of bones and air. representative of such organs include, without limitation, a breast, a tongue and a testicle.

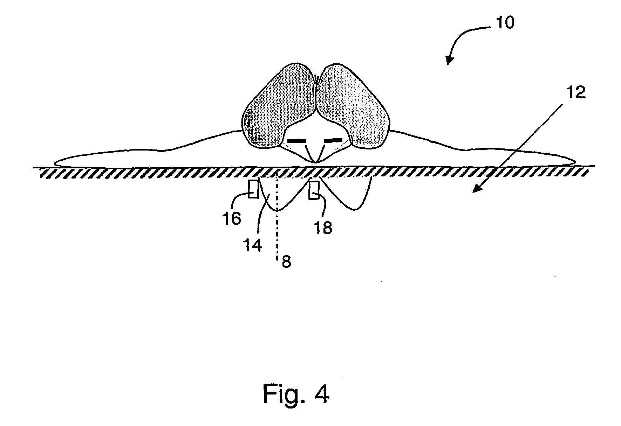

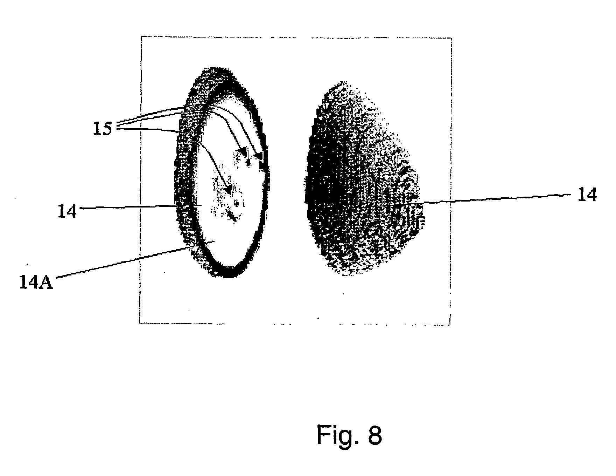

Figure 4 schematically illustrates a relevant geometry for in which the region is a breast **14.** Breast **14** of a body **10,** can be immersed in a tissue coupling medium **12,** such as water. Ultrasound transducers **16** and **18** are positioned on opposite sides of breast **14,** and preferably perform a rotary motion about a longitudinal axis **8** of breast **14.** It will be appreciated that when breast **14** and transducers **16** and **18** are immersed in water, the transducers need not be directly against the breast. However, where a gel is used as medium **12,** direct contact is desired. A representative example of a three-dimensional phantom reconstruction of breast **14** is provided hereinunder (see Figure 8 in the Example section that follows).

Additionally, the TUCT images of the present embodiments can be obtained for portions of external organs provided these portions do not contain bones or air and can be inserted between the ultrasound devices. These portions typically include sufficient amount of soft tissues such as, but not limited to, the fatty tissue in the upper thighs or hips, or the soft tissue in the armpit. In these embodiments, the TUCT images are acquired by lifting the soft tissue into the interspace between the ultrasound devices.

There are, however, regions in the body which can not be imaged via TUCT using only extracorporeal devices due to scattering and reflections from bones and air which may be present between the transmitter and the receiver.

The present embodiments provide more than one solution to this problem.

In one preferred embodiment, the TUCT is performed during open surgery in which case the organ to be imaged can be accessed using the TUCT system (*e.g.*, system **300** above). This embodiment is particularly useful for imaging internal organs which are both accessible and movable by the surgeon during open surgery. In cases of tumors in the liver (adenomas, hepatoma, *etc.*), for example, during open surgery the surgeon positions the ultrasound devices on both sides of the liver and acquires an image of the internal part of the liver to determine locations of pathologies such as tumors therein. Once the location(s) are determined, the surgeon can focus the HIFU on the tumor as detailed above, and destroy the tumor by ablation or cavitation. It is recognized that as the liver is an extremely bloody organ, the ability of destroying tumors in the liver without invading the liver's tissue is of utmost importance. Furthermore, in extreme cases, a portion of the liver containing an untreatable amount of tumors can be removed, while the remaining portion which contains fewer tumors (*e.g.*, metastases) can be imaged via TUCT and the tumors therein can be destroyed by HIFU.

The above procedure can be performed also for other organs such as a kidney, colon, stomach or pancreas. In case the stomach contains gases, which may cause reflections or scattering of acoustic waves, the stomach can be filled with fluids prior to the procedure.

Another organ which can be imaged in various exemplary embodiments of the invention is the brain. The brain can contain many types of tumor which can be diagnosed or located, according to the teaching of the present embodiments. Representative examples include, without limitation, primary benign tumors such as meningioma, primary malignant tumors such as glyoblastoma or astrocytoma, and any malignant metastasis to the brain from any organ such as colon, breast, testis and the like.

This can be achieved, for example, during open brain surgery. In this embodiment, a portion of the cranium is removed and the ultrasound transducers are inserted, preferably in a 180° arrangement, between the brain and the remaining portion of the cranium. Alternatively, opposite parts of the brain can be exposed, by drilling or removing opposite portions of the cranium, and the ultrasound devices can be brought to engage the exposed parts. A TUCT image of the brain can then be generated using the engaging ultrasound devices as further detailed above. If the brain contains pathologies such as tumors, the pathologies can be destroyed or at least partially damaged, for example, by exposing an additional part of the brain tissue and engaging the exposed part with a HIFU device to damage the pathologies by ablation or cavitation.

TUCT images can also generated by minimally invasive procedures in which intracorporeal and extracorporeal ultrasound devices are employed.

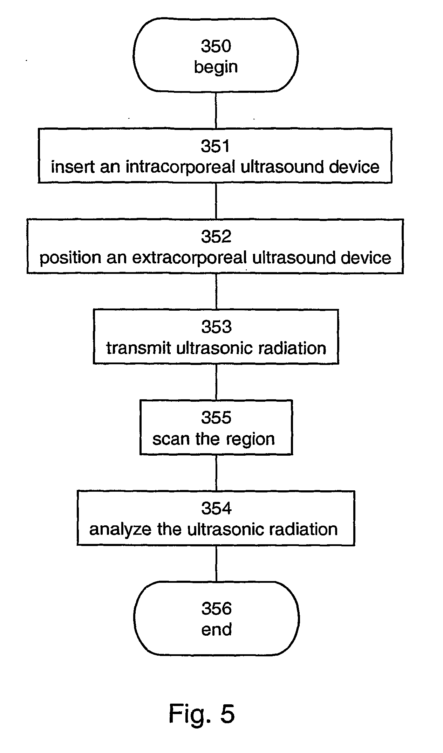

Reference is now made to Figure 5, which is a flowchart diagram of a method for imaging a region of a subject, according to a preferred embodiment of the present invention. The method begins at step **350** and continues to step **351** in which an intracorporeal ultrasound device is inserted to the subject. The intracorporeal ultrasound device is preferably inserted endoscopically by mounting the device on a suitable transport mechanism, such as, but not limited to, an endoscopic probe or a catheter. The intracorporeal ultrasound device is preferably flexible so as to facilitate its endoscopic insertion. Additionally and preferably the intracorporeal ultrasound device is sizewise and geometrically compatible with the internal cavities of the subject so as to minimize discomfort of the subject during the non-invasive in vivo examination. Thus, the intracorporeal ultrasound device is preferably adapted for transrectal, transurethral, transvaginal or transesophageal examination.

The method proceeds to step **352** in which an extracorporeal ultrasound device is positioned opposite to the intracorporeal ultrasound device, in a transmitter-receiver configuration (see, *e.g.*, the configuration exemplified in Figure 3), such that at least a portion of the region is interposed between the intracorporeal and extracorporeal ultrasound devices.

As stated above, the ultrasound devices preferably oppose one another in a 180° arrangement by connecting the devices by a bridge, or by employing an appropriate calibration procedure. Additionally, the positioning of the devices can be done by activating the extracorporeal ultrasound device in a pulse-echo mode and displaying an image of the internal region including the intracorporeal ultrasound device. Being a solid object, the intracorporeal ultrasound device has a sufficiently high acoustic reflection coefficient, and its location within the image can be determined even for the relatively low resolution of the pulse-echo ultrasound image. The extracorporeal ultrasound device can then be moved by the operator, for example, until the intracorporeal ultrasound device is seen at the center of the pulse-echo ultrasound image.

Once the intracorporeal and extracorporeal ultrasound devices are positioned, the method continues to step **353** in which ultrasonic radiation is transmitted through the region. Preferably, but not obligatorily, the ultrasonic radiation is transmitted from the intracorporeal ultrasound device and received by the extracorporeal ultrasound device. Alternatively, the ultrasonic radiation can be transmitted from the extracorporeal ultrasound device and received by the intracorporeal ultrasound device. Still alternatively, both devices can serve for transmitting and receiving ultrasonic radiation.

The method continue to step **354** in which the region is scanned using the intracorporeal ultrasound device, the extracorporeal ultrasound device or both the intracorporeal and extracorporeal ultrasound devices. The scanning can be done by rotating the extracorporeal ultrasound device about the intracorporeal ultrasound device. When the devices are connected by a bridge, at any point along the motion path of the extracorporeal ultrasound device, the two devices oppose each other. When the devices are not connected by a bridge, the calibration or the pulse-echo imaging procedure is preferably repeated for each displacement of the devices.

According to a preferred embodiment the method continues to step **355** in which the ultrasonic radiation is analyzed so as to generate an image of the region by TUCT.

The method ends at step **356.**

The above method can be used for imaging many internal regions of the human body.

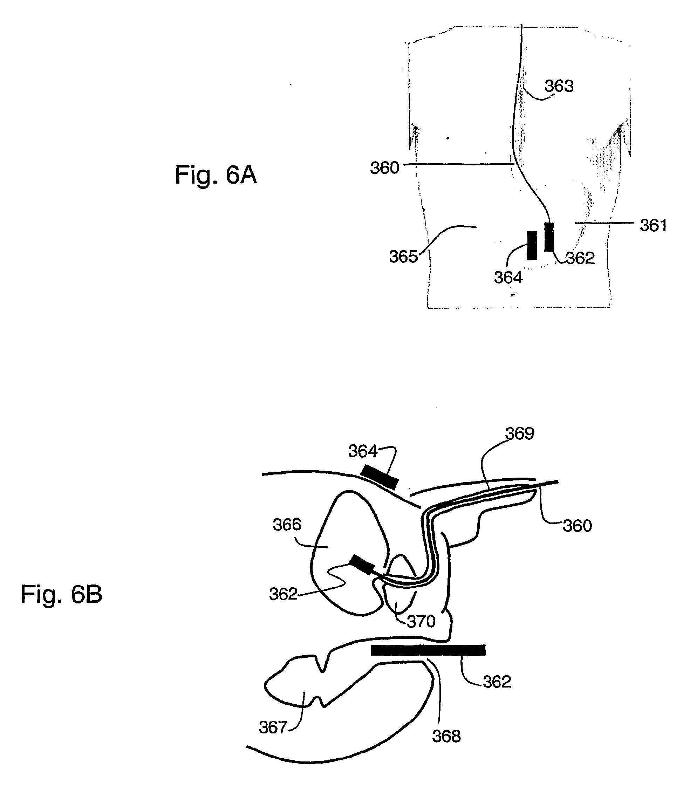

Reference is now made to Figure 6A, which is a schematic illustration of an embodiment in which the intracorporeal and extracorporeal ultrasound devices are used for imaging the stomach by TUCT. Shown in Figure 6A is the esophagus **360** and the stomach **361** (image source: National Library of Medicine (NLM) web site). Also shown are the intracorporeal ultrasound device **362,** is inserted through esophagus **360** by a catheter **363** and positioned in stomach **361.** Extracorporeal ultrasound device **364** is positioned externally on the upper abdomen **365,** opposite to device **363.** In operation mode, ultrasonic radiation is transmitted through the stomach to provide a TUCT image thereof as further detailed above. This embodiment can be used for imaging benign tumors such as Leomyoma, or malignant tumors such as carcinoma or lymphoma.

The ability to insert the intracorporeal ultrasound device through the esophagus allows the operator to obtain TUCT images of the esophagus itself, thereby to locate pathologies, such as the carcinoma of the esophagus, thereon. In this embodiment, both the devices are positioned such that the ultrasonic energy is transmitted through the gap between two ribs. Such positioning can be performed, for example, by operating the extracorporeal ultrasound device in the aforementioned pulse-echo mode and displaying an image of the internal region including the ribs and the intracorporeal ultrasound device. The devices can then be brought to the desired location under the pulse-echo display.

Reference is now made to Figure 6B, which is a schematic illustration of an embodiment in which the intracorporeal and extracorporeal ultrasound devices are used for imaging the prostate or bladder by TUCT. Shown in Figure 6B are the rectum **367,** the bladder **366,** the prostate **370** and the urethra **369.** In the present embodiments, intracorporeal ultrasound device **362** can be inserted into through the anus **368** into the rectum **367,** or through the urethra **369.** When device **362** is inserted through the urethra it can be used for imaging the prostate, in which case device **362** is positioned near the prostate, or the bladder, in which case device **362** is inserted into the bladder as shown in Figure 6b. Extracorporeal ultrasound device **364** can then be positioned on the lower external abdomen, and a TUCT image of the prostate or the bladder can be obtained.

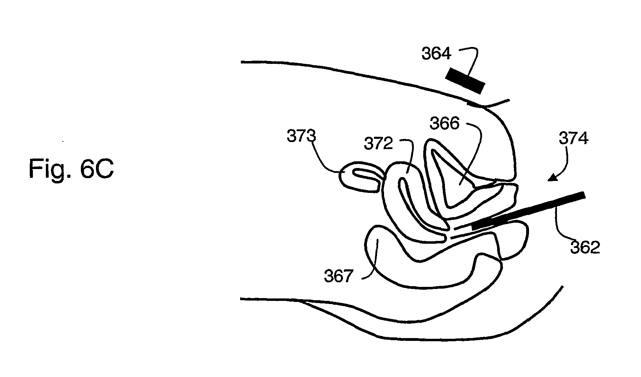

Reference is now made to Figure 6C, which is a schematic illustration of an embodiment in which the intracorporeal and extracorporeal ultrasound devices are used for imaging the uterus, bladder or ovary by TUCT. Shown in Figure 6C are the rectum **367,** the bladder **366,** the uterus **372** and the ovary **373.** In the present embodiments, device **362** can be inserted into through the vagina **374.** Device **362** can be mounted on a catheter and can be inserted into the uterus. Extracorporeal ultrasound device **364** can then be positioned on the mid external abdomen, and a TUCT image of the uterus, bladder or ovary can be obtained. This embodiment imaging can be used for locating or diagnosing polyps in the uterus or bladder. Additionally this embodiment can be used for locating or diagnosing benign tumors in the uterus (*e.g.*, myomas) or any malignant tumors therein. For the ovary, this embodiment can be used for imaging any primary or secondary malignant tumors therein.

The imaging technique of the present embodiments therefore enjoys many properties, such as non hazardous radiation, cost-effectiveness, simplicity and compactness.

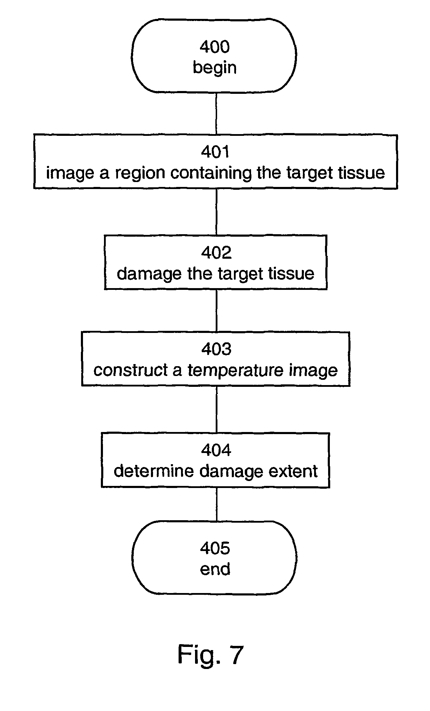

The present embodiments successfully provide a TUCT-guided method suitable for damaging a target tissue. The method comprises the following method steps which are illustrated in the flowchart of Figure 7. The method begins at step **400** and continues to step **401** in which the region is imaged by TUCT as further detailed hereinabove. The method proceeds to step **402** in which the target tissue is damaged by an effective amount of damaging radiation. The damaging radiation can be applied by any radiation system which generates or transmits radiation capable of damaging the tissue. Thus, the damaging radiation can be HIFU radiation generated or transmitted by a HIFU system, microwave radiation generated or transmitted by microwave ablation (MWA) system, radiofrequency radiation generated or transmitted by a radiofrequency ablation system, and the like. In an additional step of the method, designated by Block **403** a temperature image of the region is constructed as further detailed hereinabove. Preferably, but not obligatorily the method continues to step **404** in which the damage extent is determined as further detailed hereinabove. The method can comprise other steps, such as, but not limited to, administration of contrast agent, post treatment imaging and damage assessment, as further detailed hereinabove. Such additional steps are omitted from the flowchart diagram for clarity of presentation.

The method ends at step **405**.

Following is a non-exhaustive list of acoustic properties and associated point projections which can be used in any embodiment in which TUCT imaging is employed.

In one embodiment, the acoustic property is the refractive index, which is proportional to the reciprocal of the speed of sound, C, in the medium. The parameter associated with the refractive index is commonly known as the time of flight. Denoting the transmission time (at point $S_1$) by $t_0$ and the receiving time (at point $S_2$) by $t_1$, the time of flight, defined as the difference $t_1$ - to, can be used as a point projection $p(x_0,z_0)$ of the refractive index:

$$p(x_0, z_0) = t_1 - t_0 = \int_{y=y_0}^{y=y_0+L} 1/C(x_0, y, z_0) \cdot dy \qquad \text{(EQ. 5)}$$

In another embodiment, the acoustic property is the attenuation coefficient $\mu$. When this property is used, the point projection can be the natural logarithm of the ratio between the amplitude of the received wave, $A_2$, and the amplitude of the transmitted wave, $A_1$:

$$p(x_0, z_0) = ln\left(\frac{A2}{A1}\right) = -\int_{y=y_0}^{y=y_0+L} \mu(x_0, y, z_0) \cdot dy \qquad \text{(EQ. 6)}$$

In an additional embodiment, the acoustic property is the derivative of the speed of sound $C$ with respect to the frequency. This property is associated with a parameter, referred to herein as "frequency-dependent velocity dispersion", which can also be used as the point projection:

$$p(x_0, z_0) = \int_{y=y_0}^{y=y_0+L} \left[\frac{\partial C(x_0, y, z_0, f)}{\partial f}\right] \cdot dy \qquad \text{(EQ. 7)}$$

The present Inventors have found the frequency-dependent velocity dispersion parameter to be useful for imaging of ablated region, for example, as seen in conjunction with Figure 12B, hereinbelow.

In still another embodiment, the definition of point projection is based on the so-called "vibro-acoustic phenomenon" (Mostafa Fatemi, Lester E. Wold, Azra Alizad, and James F. Greenleaf, Vibro-Acoustic Tissue Mammography, IEEE Transactions On Medical Imaging, 21, 1, 2001) which is related to the aforementioned nonlinear wave phenomenon. In this embodiment, two waves, at frequencies $f_1$ and $f_2$, are transmitted and the point projection is calculated as:

$$p(x_0, z_0) = \int_{y=y0}^{y=y0+L} A[f_h(x, y, z)] dy, \qquad \text{(EQ. 8)}$$

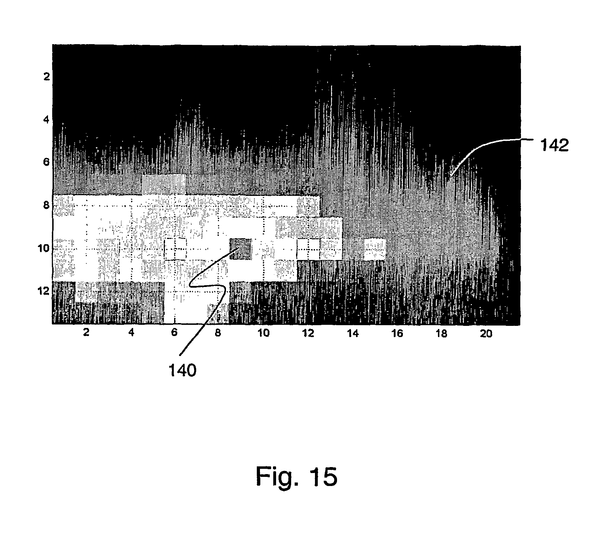

where $f_h$ is a harmonic combination defined above ($f_h = mf_1 + nf_2$) and $A$ is a property (*e.g.*, energy, pressure amplitude) of the received wave at frequency $f_h$. In various exemplary embodiments of the invention $f_h = \Delta f = |f2 - f_1|$. The present Inventors have found that the use of TUCT imaging with the projection defined in Equation 8 is useful for determining the focal region, see, *e.g.*, Figure 15 below and the accompanying description.

In yet another embodiment, also related to nonlinear wave phenomena, the acoustical property is defined using harmonics generated during the propagation of the acoustic wave in the nonlinear medium. The harmonics of a wave of frequency *f* are *n-f*, where *n* is an integer larger than 1 (*i.e., n* = 2 , 3...). These harmonics have shorter wave lengths and can be used for both producing a new type of contrast and improved resolution. The corresponding point projection is given by:

$$p(x_0, z_0) = \int_{y=y_0}^{y=y_0+L} A[n \cdot f(x,y,z)] \cdot dy \qquad\qquad (EQ.\ 9)$$

where *A* is a property (*e.g*, energy, pressure amplitude) of the high harmonic wave.

Additional objects, advantages and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

## EXAMPLES

Reference is now made to the following examples, which together with the above descriptions illustrate the invention in a non limiting fashion.

### *EXAMPLE 1*

### *TUCT image*

Figure 8 illustrates a three-dimensional phantom reconstruction of breast **14** based on attenuation-coefficient imaging, as obtained by the spiral transmission ultrasound computerized tomography (SUCT) and reported in a paper by Azhari H. and Sazbon D., entitled "Volumetric imaging using spiral ultrasonic computed tomography", published in Radiology, 1999, 212(1):270-275. A three-dimensional computerized reconstruction section **14A** was virtually cut at about 10 mm from the base of breast **14**, to depict target masses **15** simulating abnormal tissue. Related work in the field includes that of Greenleaf, *et al.* [Greenleaf James F., and Bahn, Robert C., "CLINICAL IMAGING WITH TRANSMISSIVE ULTRASONIC COMPUTERIZED TOMOGRAPHY," IEEE Trans, Biomed Eng, v BME-28, n 2, Feb 1981, p 177-185], which describes transmission ultrasound computer-assisted tomography for detection and diagnosis of cancer in the breast, and Jago, et al. [Jago, J. R., Whittingham, T. A., "Practical system for the application of ultrasound computed tomography to medical imaging," IEE CONF PUBL, the International Conference on Acoustic Sensing and Imaging CONFERENCE LOCATION- London, UK, March, 29-30, 1993, no. 369, pp. 257-265], which outline the progress made towards the realization of practical systems for medical reflection and transmission ultrasound computed tomography (UCT) imaging.

### *EXAMPLE 2*

### *TUCT System*

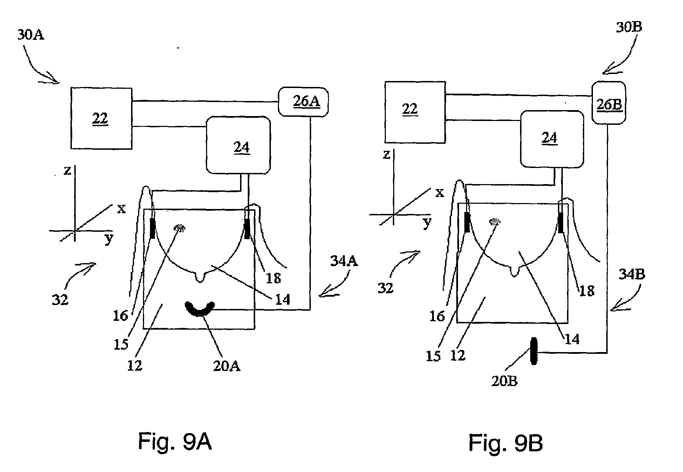

Figures 9A-C schematically illustrate configurations **30A** and **30B**, for producing a temperature image (thermal map) using a TUCT system **32**, according to various exemplary embodiments of the invention.

As shown in Figure 9A, configuration **30A** comprises a TUCT system **32**, which comprises ultrasonic transducers or transducer arrays **16** and **18**. Transducers **16** and **18** are in communication with an imaging unit **24**. Data processor **22** is further operative to perform data analysis and display, by graphical means, by printout, or by other means. It will be appreciated that data processor **22** may be integrated with imaging unit **24**, so as to form a single unit. Preferably, transducers **16** and **18** and organ **14** (shown in Figures 9A-B as a breast) are immersed in or applied with tissue coupling medium **12**.

Additionally, a surgical-procedure unit, which is operative to heat a portion of the tissue, for example, a HIFU system **34A**, is preferably also in communication with data processor **22**, which may operate system **34A** automatically. HIFU system **34A** is adapted to destroy a tumor **15** by ablation or cavitation, and comprises an HTFU operating unit **26A** and an HIFU transducer or transducer array **20A**, in communication with organ **14**, via tissue coupling medium **12**. Transducer **20A** is preferably focused on tumor **15**, such that peak ablation temperatures, produced by transducer **20A**, occur within tumor **15**. Typical ablation temperatures within and around tumor **15** are from 55 to about 60 degrees centigrade so as to achieve ablation of the tumor substantially without scorching the surrounding tissue.

In accordance with the present embodiments, TUCT system **32** can be used for at least one of: (i) providing three-dimensional images of organ **14** for locating and marking the spatial coordinates of the target tissue; (ii) locating the spatial coordinates of the HIFU focal region; (iii) aiming and focusing the HIFU system **34A** on the target tissue according to the above information; (iv) obtaining a temperature image (thermal map) of organ **14,** during the damaging procedure of **34A**; and (v) providing an image of the ablated region after the procedure, for damage assessment.

As shown in Figure 9B, configuration **30B** is similarly constructed, but includes a microwave ablation (MWA) system **34B**, in place of HIFU system **34A** (Figure 9A). MWA ablation system **34B** is adapted to destroy tumor **15** by ablation, and includes a MWA operating unit **26B** and an MWA transmitter **20B**, which may be located outside medium **12**. MWA transmitter 20B is preferably focused on tumor **15**, such that the peak ablation temperatures, produced by MWA transmitter **20B**, occur within tumor **15**. Similarly to the above, the typical ablation temperatures are from about 55 to about 60 degrees centigrade.

In accordance with the present embodiments, TUCT system **32** can be used for at least one of: (i) focusing MWA ablation system **34B** on tumor **15**; (ii) obtaining a temperature image (thermal map) of organ 14, during the thermal ablation by MWA ablation system **34B**; and; (iii) providing post treatment functional images for damage assessment.

It will be appreciated that other invasive, noninvasive or minimally invasive surgical-procedure systems may be employed for ablation or cryosurgery, and in general, TUCT system **32** may be used both for guiding the surgical procedure to a target, for example, tumor 15, and for obtaining a temperature image (thermal map) of organ 14, as a consequence of the surgical procedure.

In accordance with the present embodiment, a method of employing TUCT system **32** for obtaining a temperature image (thermal map) of organ **14**, as a consequence of a temperature-related surgical procedure, is as follows: (i) employing TUCT system **32** for obtaining the reversible transform of a reference image of an acoustic property, for example, high harmonic energy, in two or three dimensions, prior to the temperature-related surgical procedure; (ii) obtaining the corresponding reference images by calculating the inverse reversible transform of the reference image, via data processor **22**; (iii) performing the temperature-related surgical procedure, for example, via HIFU system **34A** (Figure 9A), MWA ablation system **34B** (Figure 9B) or another system; (iv) employing TUCT system **32**, during the temperature-related surgical procedure, for obtaining the reversible transform of intermediate images of the corresponding acoustic property; (v) subtracting the reversible transform of the intermediate images from the reversible transform of the reference image, to obtain the reversible transform of the changes resulting from the temperature-related surgical procedure; (vi) calculating the inverse reversible transform of the subtraction data and constructing temperature images, corresponding to the intermediate images of the corresponding acoustic property.

The aforementioned method may be used to monitor the temperature profile, as a consequence of the temperature-related surgical procedure, to ensure both that ablation took place at the desired location, and that ablation temperatures were within a desired range.

A method of employing TUCT system 32 for focusing a temperature-related surgical procedure on tumor **15** includes the following steps: (i) employing TUCT system **32** and data processor **22** for obtaining a pre-surgical-referenced image, in two or three dimensions; (ii) using the reference image and data processor **22**, defining the spatial coordinates of the target tissue; (iii) performing a low-power mockup surgical procedure, for example, via HIFU system **34A** (Figure 9A), with two or more waves simultaneously one at a frequency $f_1$ and another at a frequency $f_2 = f_1 + \Delta f$, exploiting nonlinear wave phenomena and using the nonlinear wave phenomena as described in conjunction with Equation 8, hereinabove; (iv) employing TUCT system **32**, to obtain the reversible transform for the nonlinear wave phenomena; (v) using data processor **22**, calculating the corresponding inverse reversible transform, in two or three dimensions, and obtaining an image of the HIFU focal region, where peak ablation temperatures would be during ablation (an actual temperature rise need not take place when identifying the location of the focal region, because the mockup procedure is a low power procedure); (vi) registering the spatial coordinates of the focal region and refocusing the HIFU at the target, within tumor **15**; where necessary the above steps are repeated until the focal region is precisely located at the target, within tumor 15; and (vii) activating the HIFU at full power to obtain tissue ablation of the target.

When a MWA ablation system **34B** (Figure 9B) or an equivalent system is employed, a small temperature rise is obtained during the mockup activation and the focal region is detected by temperature imaging as follows: (i) employing TUCT system **32** for obtaining a pre-surgical-procedure reference reversible transform of one or more acoustic properties; (ii) using data processor **22**, calculating the inverse reversible transform and reconstruct the corresponding reference image; (iii) applying a low-power, mockup surgical procedure; (iv) employing TUCT system **32**, obtaining a post-mockup-surgical-procedure reversible transform of that one or more acoustic properties; (v) subtracting the reversible transform of the post-mockup-surgical-procedure image from the reversible transform of the reference image, to obtain the reversible transform of the low-power, mockup surgical procedure effect; (vi) calculating the inverse of the subtraction reversible transform, reconstructing a temperature image, resulting from the mockup surgical procedure; (vii) in the obtained reconstruction, locate the focal region, defined as the point at which the temperature is highest; if needed, the aim of the surgical-procedure system is corrected and steps (iii)-(viii) are repeated, until a desired temperature profile is reached at the target, within tumor **15**.

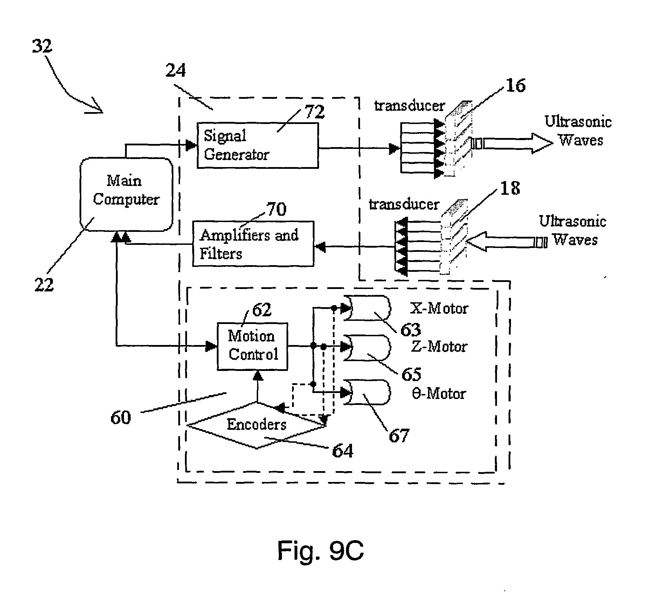

Figure 9C schematically illustrates a block diagram for TUCT system **32**, according to a preferred embodiment of the present invention. TUCT system **32** includes transmitting and receiving transducers **16** and **18**, respectively, adapted for motion, for example, along the x direction, circumferential direction θ and optionally z direction. Transducers **16** and **18** are in communication with imager **24**. Data processor **22** is similarly in communication with imager **24**. Imager **24** comprises a motion unit **60**, having a motion control **62**, an encoder **64**, and *x, z,* and θ motors respectively designated **63**, **65** and **67**. The motors receive instructions from motion control unit **62**.

Preferably, encoder **64** receives information from motors **63**, **65** and **67**, regarding the locations of transducers **16** and **18**, and informs motion control unit **62.** Based on that information, motion control unit **62** determines the next incremental course of motion.

Additionally, imager **24** comprises an amplifies-and-filters unit **70** for receiving signals from receiving transducer **18**, and a signal generator **72**, for generating signals to transmitting transducer **16**.

Preferably, the data are processed by data processor **22**, which may also control and operate system **32**.

*EXAMPLE 3*

*Configuration for HIFU Treatment of a Breast*

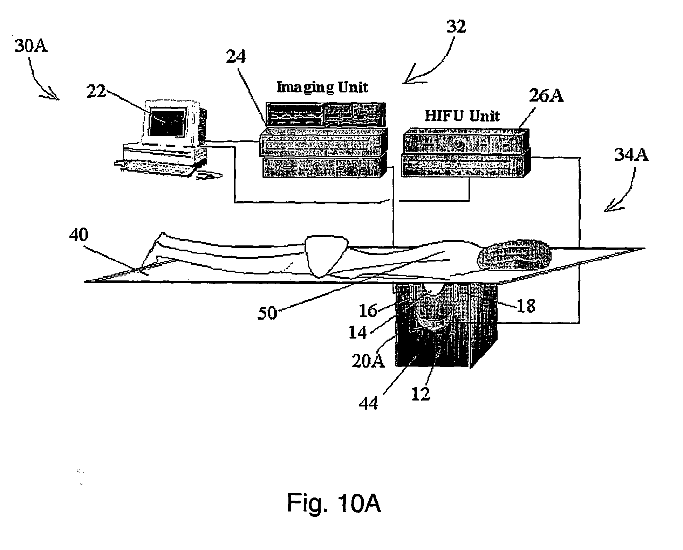

Figures 10A-C schematically illustrate configuration **30A** of Example 2 (see Figure 9A), designed for image guided HIFU treatment of a woman's breast, in accordance with various exemplary embodiments of the invention.

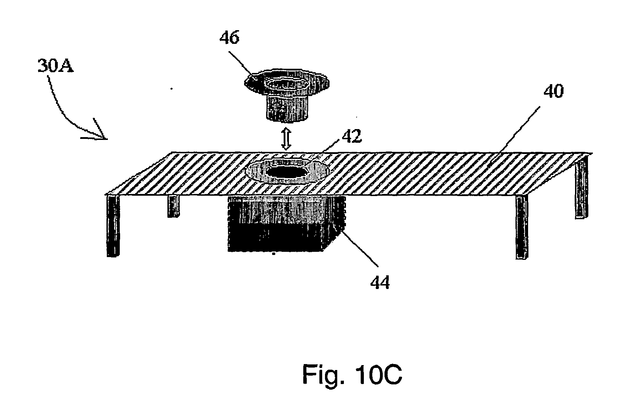

As shown in Figures 10A and 4C, a woman **50** lies prone on bed **40**, with her breast **14** inserted through hole **42** and sleeve **46** (Figure 10C) into water tank **44**, where both ultrasound imaging and ultrasound ablation are performed, under water.

As seen in Figures 10A and 4C, configuration **30A** includes a special bed **40**, which defines a hole **42**, into which breast **14** (Figures 9A-B) is to fit. Hole **42** is in communication with a water tank **44**. Preferably a removable, washable or disposable sleeve **46** is employed for hygienic purposes.

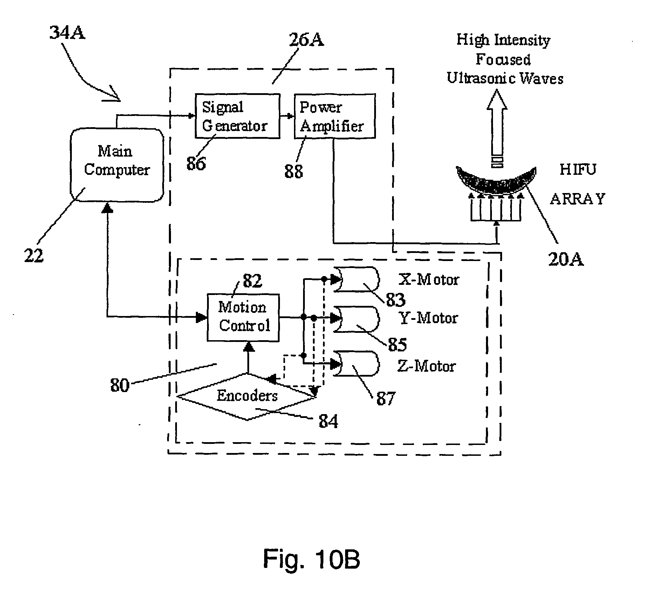

Figures 10A and 10B further illustrate HIFU system **34A**, comprising HIFU operating unit **26A** and an HIFU transducer or transducer array **20A**, and TUCT system **32**, comprising transducers **16** and **18**, imaging unit **24**, and data processor **22**.

Figure 10B schematically illustrates a block diagram for HIFU system **34A,** in accordance with various exemplary embodiments of the invention. HIFU system **34A** includes HIFU operating unit **26A** and HIFU transducer or transducer array **20A,** adapted for motion along the $x$, $y$ and $z$ axes, by motors **83, 85** and **87.** Thus, HIFU operating unit **26A** includes a motion unit **80,** having a motion control unit **82** and an encoder **84,** and $x$, $y$, and $z$ motors, **83, 85** and **87,** where the motors receiving instructions from motion control unit **82.**

Additionally, HIFU operating unit **26A** comprises a signal generator **86** and an amplifier **88,** in communication with HIFU transducer or transducer array **20A**. HIFU system 34A is preferably controlled and operated by data processor **22.**

*EXAMPLE 4*

*Temperature Image by TUCT*

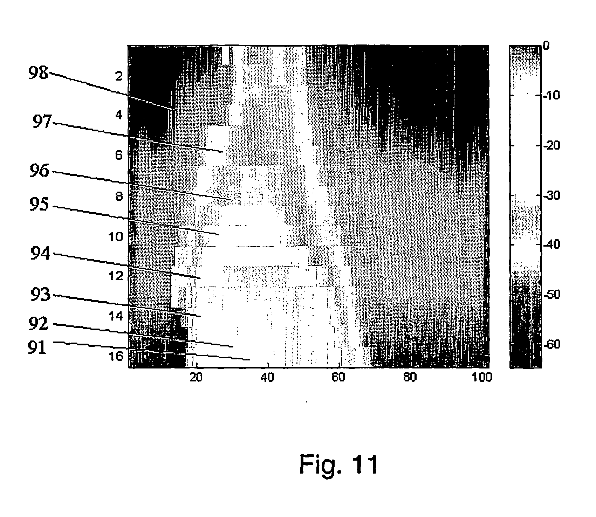

Figure 11 illustrates a temperature image as obtained by TUCT system **32**, according to a preferred embodiment of the present invention. The temperature image is of an agar phantom heated by HIFU system **34A**, operated at 11 watts and at 3 Mhz. Each unit on the color-map scale corresponds to a temperature increment of about 0.3 °C, where maximal temperature (dark red color) was about 21 °C above the water tank temperature. Seven temperature zones are observed: 91, 92, 93, 94, 95, 96, 97 and 98, with zone 91 being the hottest and zone 98 being about 5 °C above water-tank temperature.

*EXAMPLE 5*

*TUCT Images Using Frequency Dependent Velocity Dispersion*

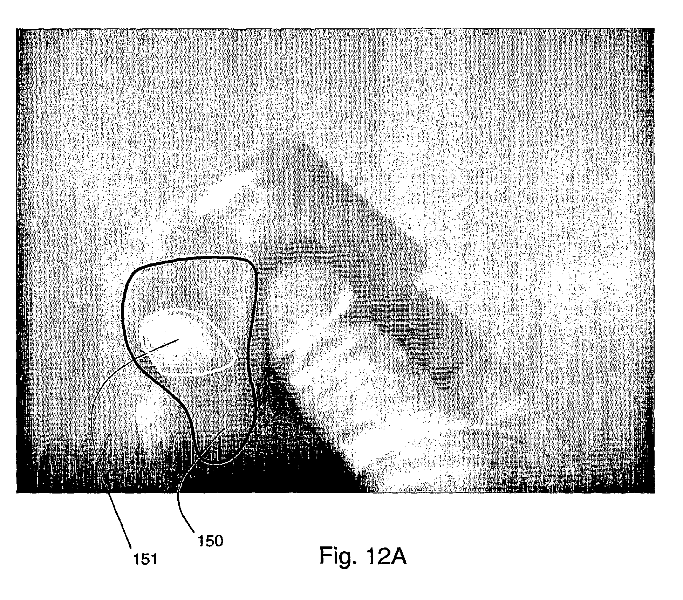

Figures 12A-C depict a photo (Figure 12A) and TUCT images (Figures 12B-C) of an in-vitro tissue specimen **150** with a HIFU ablated region **151**. In-vitro specimen **150** is a turkey breast specimen, heated by a HIFU system. Specimen **150** was cut approximately at the plane where the TUCT images depicted in Figures 12B and 12C were obtained. The TUCT images were acquired few minutes after the temperature-related surgical procedure, for verification, to ensure the success of the treatment. Figure 12B shows the TUCT image obtained using the frequency-dependent-velocity-dispersion method (see Equation 7 hereinabove) and Figure 12C shows the TUCT image obtained using the time-of-flight method.

As shown in Figure 12A the color of ablated tissue **151** is white, while the unablated portion of specimen **150** has retained its original pink color. The ablated region is depicted in Figure 12B as a pale region surrounded by a red edge. Thus, ablated region **151** is clearly identified. In other words, changes in speed dispersion (i.e. the change in the speed of sound as a function of frequency) match the shape and location of ablated region **151.**

On the other hand, in Figure 12C, obtained using the time-of-flight method, the image obtained at the same time and same plane does not provide a clear definition of ablated region **151**.

## EXAMPLE 6

### TUCT Images Using Harmonics

In accordance with various exemplary embodiments of the invention, improved resolution may be obtained by the use of higher harmonics. Where a transmitted wave has a frequency $f$, the received wave has a multiple series of higher-harmonic components, for example, at frequencies $2f$, $3f$, $4f$, and so on. Because the attenuation coefficient increases with frequency, higher harmonic waves are much weaker than the first harmonic, yet, they may be used to improve the resolution which is inversely proportional to the wavelength (and which is shorter for higher harmonics).

Additionally, improved image resolution, with higher harmonics, may be employed when several frequencies are used in transmission. For example, where a transmitted wave comprises two frequencies $f_1$ and $f_2$, the received wave has several linear combinations of these two frequencies, such as $m f_1 \pm n f_2$, where $m$ and $n$, as stated above, are integers. These higher harmonics combination can be used to produce images of the tissues. Again, since the resolution is inversely proportional to the wavelength, these offer higher resolution than the original wave.

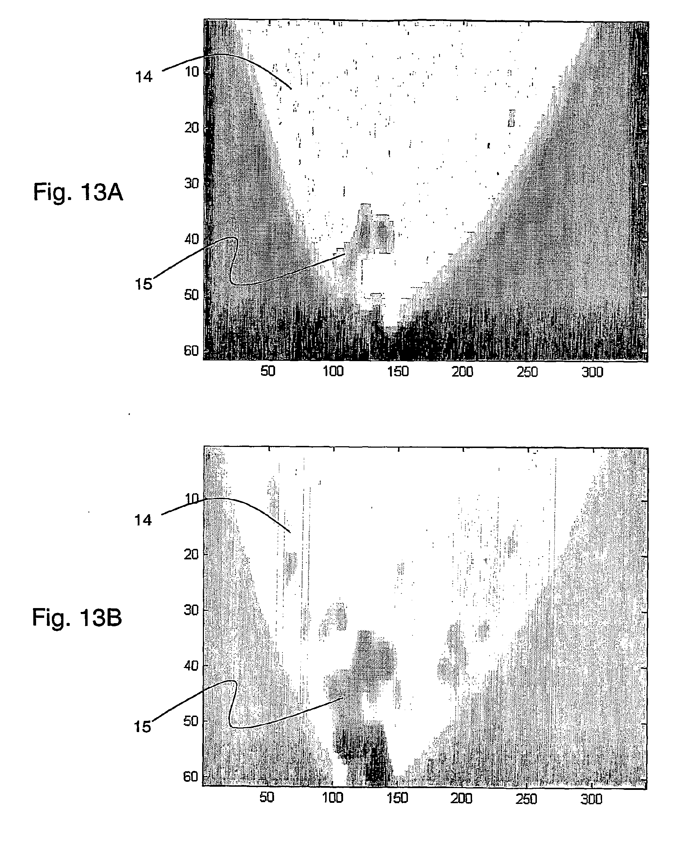

Referring further to the drawings, Figures 13A-B depict a lateral view of an actual in-vivo scan of a breast **14** with papiloma growth **15**, as obtained by TUCT system **32**, using third harmonic band imaging (Figure 13A) and first harmonic band imaging (Figure 13B). As shown, Figure 13A of the third harmonic band imaging has better resolution that Figure 13B of the first harmonic band imaging.

### Example 7

### Use of Harmonics as Spectral Signature

It has been theoretically hypnotized and experimentally uncovered by the present Inventors that the harmonics can further be exploited for providing "spectral signatures" for the different tissue types. In other words, different tissue types, such as a healthy tissue, a cancerous tissue, a bone tissue or an ablated tissue, may each have a spectral signature in a form of a characteristic set of harmonics $f_h$, which can identify it.

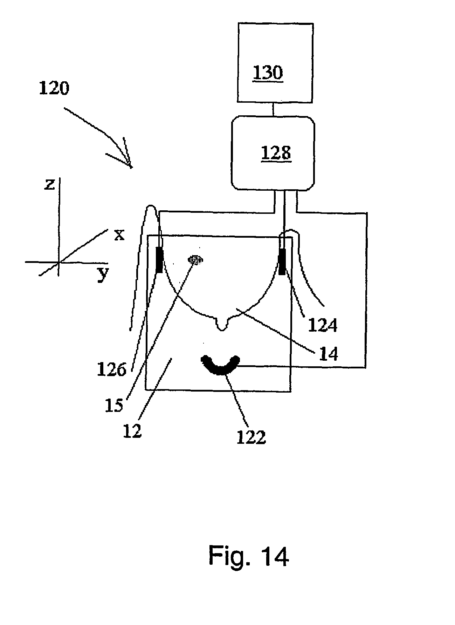

Referring further to the drawings, Figure 14 schematically illustrates another configuration for a high intensity focused ultrasound (HIFU) system **120**, with built-in focusing, according to a preferred embodiment of the present invention.

HIFU system **120** comprises an HIFU transducer or transducer array **122**, in communication with organ **14**, via tissue coupling medium **12**. In accordance with the present embodiment, HIFU system **120** further comprises at least one passive transducer or transducer array **124**. Shown in Figure 14 are two passive transducers or transducer arrays **124** and **126**, but this should not be considered as limiting as any number of transducer can be used. Arrays **124** and **126** are adapted to receive waves generated in the tissue as a result of the waves transmitted by transducer or transducer array **122**.

Additionally, HIFU system **120** comprises an operating and control unit **128**, which may be connected to a data processor **130**. Alternatively, an operating and control unit **128** has a built in data processor.

According to a preferred embodiment of the present invention, HIFU transducer or transducer array **122** is adapted for producing low-power ultrasound bursts of two or more frequencies, $f_1$ and $f_2$, while transducer **124**, and possibly also transducer **126** operate as receivers. Operating and control unit **128** is adapted to receive signals from transducer **124**, and possibly also transducer **126**, and determine the focal region of transducer or transducer array **122**, based on the points of maxima observed for combinations of the frequencies or of harmonics of the frequencies (*e.g.*, $m f_1 \pm n f_2$). Thus, system **120** is dedicated for HIFU, with a dedicated mechanism for focusing.

Figure 15 depicts a focal region **140**, as identified by means of harmonic combination projection described hereinabove using two frequencies, $f_1$ and $f_2$, in accordance with various exemplary embodiments of the invention. In the present example, frequencies of 1.01 MHz and 3.14 MHz were selected for $f_1$ and $f_2$, respectively. The waves were aimed at a balloon filled with vegetable oil **142**. Transducer **124**, operating in a passive mode, that is as a receiver only, scanned the object. Focal region **140** was generated when the waves of the two frequencies interact and a new wave with a frequency which equals their sum ($f_1 + f_2 = 4.15$ MHz) is generated. It will be appreciated that additional frequencies, for example, $f_1$, $f_2$, $f_3$, may be used.

It is expected that during the life of this patent many relevant transmission-ultrasound computerized tomography and high intensity focused ultrasound systems may be developed and the scope of the terms transmission-ultrasound computerized tomography and high intensity focused ultrasound systems is intended to include all such new technologies *a priori*.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

**Claims**

1. A high intensity focused ultrasound (HIFU) system, comprising:

   a HIFU device, capable of transmitting the HIFU from a plurality of directions and at a plurality of different frequencies, and receiving ultrasonic radiation having at least one frequency other than said plurality of different frequencies; and
   a data processor, designed and constructed to determine a focal region of said HIFU based on points of maxima of the acoustic energy carried by and observed for said at least one frequency other than said plurality of different frequencies.

2. A system for damaging a target tissue, comprising:

   (a) an imaging system, for imaging a region containing the target tissue;
   (b) a high intensity focused ultrasound (HIPU) device, capable of transmitting the HIFU from a plurality of directions and at a plurality of different frequencies, and receiving ultrasonic radiation having at least one frequency other than said plurality of different frequencies; and
   (c) a data processor, designed and constructed to determine a focal region of said HIFU based on points of maxima of the acoustic energy carried by and observed for said at least one frequency other than said plurality of different frequencies.

3. The system of claim 2, wherein said imaging system comprises a transmission ultrasound computerized tomography (TUCT) system for imaging said region by TUCT.

4. The system of claim 3, wherein said TUCT system comprises an intracorporeal ultrasound device, an extracorporeal ultrasound device, and a data processor for analyzing ultrasonic radiation transmitted between said intracorporeal ultrasound device and said extracorporeal ultrasound device so as to generate an image of the region.

5. The system of claim 2, wherein said imaging system and said HIFU device are designed and constructed to operate substantially contemporaneously.

6. The system of claim 2, wherein said damaging comprises ablation.

7. The system of claim 2, wherein said damaging comprises cavitation.

8. The system of claim 4, wherein said ultrasonic radiation is transmitted from said intracorporeal ultrasound device and received by said extracorporeal ultrasound device.

9. The system of claim 4, wherein said ultrasonic radiation is transmitted from said extracorpreal ultrasound device and received by said intracorporeal ultrasound device.

10. The system of claim 9, wherein said ultrasonic radiation is transmitted from said intracorporeal ultrasound device and received by said extracorporeal ultrasound device.

11. The system of claim 4, wherein said intracorporeal ultrasound device is adapted to be inserted through the anus.

12. The system of claim 4, wherein said intracorporeal ultrasound device is adapted to be inserted through the vagina.

13. The system of claim 4, wherein said intracorporeal ultrasound device is adapted to be inserted through the urethra.

14. The system of claim 4, wherein said intracorporeal ultrasound device is adapted to be inserted through the esophagus.

15. The system of claim 4, wherein said intracorporeal ultrasound device is mounted on a transport mechanism.

16. The system of claim 15, wherein said transport mechanism is selected from the group consisting of an endoscopic probe and a catheter.

17. The system of claim 2, wherein said imaging system is operable to employ pulse-echo imaging.

18. The system of claim 2, wherein said imaging system is operable to employ inverse scattering imaging.

19. The system of claim 2, wherein said imaging system is operable to employ magnetic resonance imaging.

20. The system of claim 2, wherein said imaging system is operable to employ thermoacoustic computerized tomography.

21. The system of claim 3-5, wherein said TUCT comprises analysis of frequency harmonics.

22. The system of claim 3-5, wherein said TUCT comprises analysis of frequency combinations.

23. The system of claim 3-5 wherein said TUCT comprises analysis of frequency harmonic combinations.

24. The system of claim 3-5 wherein said TUCT is effected by spiral scanning.

25. The system of claim 3-5 wherein said TUCT comprises analysis of time-of-flight.

26. The system of claim 3-5 wherein said TUCT comprises analysis of phase shift.

27. The system of claim 3-5 wherein said TUCT comprises analysis of frequency-dependent velocity dispersion.

28. The system of claim 1 or 2, being non invasive.

29. The system of claim 1 or 4, being minimally invasive.

**Patentansprüche**

1. HIFU-(Hochfokussierter Ultraschall-)System, umfassend:

   ein HIFU-Gerät, das in der Lage ist, den HIFU von einer Mehrzahl von Richtungen und in einer Mehrzahl von unterschiedlichen Frequenzen zu übertragen und Ultraschallstrahlung mit zumindest einer Frequenz, die sich von der Mehrzahl von unterschiedlichen Frequenzen unterscheidet, zu empfangen; und
   einen Datenprozessor, der konzipiert und konstruiert ist, um einen Fokusbereich des HIFU auf Basis von Maximapunkten der akustischen Energie, die von der zumindest einen Frequenz, die sich von der Mehrzahl der unterschiedlichen Frequenzen unterscheidet, getragen und für diese beobachtet wird.

2. System zum Schädigen eines Zielgewebes, umfassend:

   (a) ein Bildgebungssystem zur Abbildung einer Region, die das Zielgewebe enthält;
   (b) ein HIFU-(Hochfokussierter Ultraschall-)Gerät, das in der Lage ist, den HIFU von einer Mehrzahl von Richtungen und in einer Mehrzahl von unterschiedlichen Frequenzen zu übertragen und Beschallung mit zumindest einer Frequenz, die sich von der Mehrzahl von unterschiedlichen Frequenzen unterscheidet, zu empfangen; und
   (c) einen Datenprozessor, der konzipiert und konstruiert ist, um einen Fokusbereich des HIFU auf Basis von Maximapunkten der akustischen Energie, die von der zumindest einen Frequenz, die sich von der Mehrzahl der unterschiedlichen Frequenzen unterscheidet, getragen und für diese beobachtet wird.

3. System nach Anspruch 2, wobei das Bildgebungssystem ein Ultraschall-Transmissions-Computertomographie-(UTCT-)System zur Abbildung der Region mittels UTCT umfasst.

4. System nach Anspruch 3, wobei das UTCT-System ein intrakorporales Ultraschallgerät, ein extrakorporales Ultraschallgerät und einen Datenprozessor zum Analysieren von Ultraschallstrahlung, die zwischen dem intrakorporalen Ultraschallgerät und dem extrakorporalen Ultraschallgerät übertragen wird, umfasst, um ein Bild der Region zu erzeugen.

**5.** System nach Anspruch 2, wobei das Bildgebungssystem und das HIFU-Gerät konzipiert und konstruiert sind, um im Wesentlichen gleichzeitig zu arbeiten.

**6.** System nach Anspruch 2, wobei das Schädigen Ablation umfasst.

**7.** System nach Anspruch 2, wobei das Schädigen Kavitation umfasst.

**8.** System nach Anspruch 4, wobei die Ultraschallstrahlung von dem intrakorporalen Ultraschallgerät übertragen und von dem extrakorporalen Ultraschallgerät empfangen wird.

**9.** System nach Anspruch 4, wobei die Ultraschallstrahlung von dem extrakorporalen Ultraschallgerät übertragen und von dem intrakorporalen Ultraschallgerät empfangen wird.

**10.** System nach Anspruch 9, wobei die Ultraschallstrahlung von dem intrakorporalen Ultraschallgerät übertragen und von dem extrakorporalen Ultraschallgerät empfangen wird.

**11.** System nach Anspruch 4, wobei das intrakorporate Ultraschallgerät eingerichtet ist, um durch den Anus eingeführt zu werden.

**12.** System nach Anspruch 4, wobei das intrakorporale Ultraschallgerät eingerichtet ist, um durch die Vagina eingeführt zu werden.

**13.** System nach Anspruch 4, wobei das intrakorporale Ultraschallgerät eingerichtet ist, um durch die Harnröhre eingeführt zu werden.

**14.** System nach Anspruch 4, wobei das intrakorporale Ultraschallgerät eingerichtet ist, um durch die Speiseröhre eingeführt zu werden.

**15.** System nach Anspruch 4, wobei das intrakorporale Gerät an einem Transportmechanismus angebracht ist.

**16.** System nach Anspruch 15, wobei der Transportmechanismus aus der Gruppe bestehend aus einer endoskopischen Sonde und einem Katheter ausgewählt ist.

**17.** System nach Anspruch 2, wobei das Bildgebungssystem betreibbar ist, um Puls-Echo-Bildgebung anzuwenden.

**18.** System nach Anspruch 2, wobei das Bildgebungssystem betreibbar ist, um Inverse-Streuungs-Bildgebung anzuwenden.

**19.** System nach Anspruch 2, wobei das Bildgebungssystem betreibbar ist, um Magnetresonanz-Bildgebung anzuwenden.

**20.** System nach Anspruch 2, wobei das Bildgebungssystem betreibbar ist, um thermoakustische Computertomographie anzuwenden.

**21.** System nach Anspruch 3 bis 5, wobei die UTCT eine Analyse von Frequenz-Harmonischen umfasst.

**22.** System nach Anspruch 3 bis 5, wobei die UTCT eine Analyse von Frequenzkombinationen umfasst.

**23.** System nach Anspruch 3 bis 5, wobei die UTCT eine Analyse von Kombinationen aus Frequenz-Harmonischen umfasst.

**24.** System nach Anspruch 3 bis 5, wobei die UTCT mittels Spiralabtastung erfolgt.

**25.** System nach Anspruch 3 bis 5, wobei die UTCT eine Flugzeitanalyse umfasst.

**26.** System nach Anspruch 3 bis 5, wobei die UTCT eine Phasenverschiebungsanalyse umfasst.

**27.** System nach Anspruch 3 bis 5, wobei die UTCT eine Analyse der frequenzabhängigen Geschwindigkeitsverteilung umfasst.

28. System nach Anspruch 1 oder 2, das nicht invasiv ist.

29. System nach Anspruch 1 oder 4, das minimal invasiv ist.


**Revendications**

1. Système à ultrasons focalisés de haute intensité (*High Intensity Focused Ultrasound,* HIFU), comprenant :

   - un dispositif HIFU capable de transmettre les ultrasons focalisés de haute intensité à partir d'une pluralité de direction et à une pluralité de fréquences différentes et recevant une radiation ultrasonique ayant au moins une fréquence autre que ladite pluralité de fréquences différentes ; et
   - un processeur de données conçu et réalisé afin de déterminer une zone focale desdits ultrasons focalisés de haute intensité sur la base de points de maximum de l'énergie acoustique transportée observés pour ladite au moins une fréquence autre que ladite pluralité de fréquences différentes.

2. Système destiné à endommager un tissu cible, comprenant :

   (a) un système d'imagerie pour représenter une zone contenant le tissu cible ;
   (b) un dispositif à ultrasons focalisés de haute intensité (*High Intensity Focused Ultrasound,* HIFU) capable de transmettre les ultrasons focalisés de haute intensité à partir d'une pluralité de direction et à une pluralité de fréquences différentes et recevant une radiation ultrasonique ayant au moins une fréquence autre que ladite pluralité de fréquences différentes ; et

   - un processeur de données conçu et réalisé afin de déterminer une zone focale desdits ultrasons focalisés de haute intensité sur la base de points de maximum de l'énergie acoustique transportée observés pour ladite au moins une fréquence autre que ladite pluralité de fréquences différentes.

3. Système selon la revendication 2, dans lequel ledit système d'imagerie comprend un système de tomographie informatisée par émission d'ultrasons (*Transmission Ultrasound Computerized Tomography,* TUCT) pour représenter ladite zone par voie de TUCT.

4. Système selon la revendication 3, dans lequel ledit système TUCT comprend un dispositif à ultrasons intracorporel, un dispositif à ultrasons extracorporel et un processeur de données pour analyser la radiation ultrasonique transmise entre ledit dispositif à ultrasons intracorporel et ledit dispositif à ultrasons extracorporel de façon à produire une image de la zone.

5. Système selon la revendication 2, dans lequel ledit système d'imagerie et ledit dispositif HIFU sont conçus et réalisés afin de fonctionner sensiblement simultanément.

6. Système selon la revendication 2, dans lequel ledit endommagement comprend l'ablation.

7. Système selon la revendication 2, dans lequel ledit endommagement comprend la cavitation.

8. Système selon la revendication 4, dans lequel ladite radiation ultrasonique est transmise à partir dudit dispositif à ultrasons intracorporel et reçue par ledit dispositif à ultrasons extracorporel.

9. Système selon la revendication 4, dans lequel ladite radiation ultrasonique est transmise à partir dudit dispositif à ultrasons extracorporel et reçue par ledit dispositif à ultrasons intracorporel.

10. Système selon la revendication 9, dans lequel ladite radiation ultrasonique est transmise à partir dudit dispositif à ultrasons intracorporel et reçue par ledit dispositif à ultrasons extracorporel.

11. Système selon la revendication 4, dans lequel ledit dispositif à ultrasons intracorporel est adapté pour être inséré par le biais de l'anus.

**12.** Système selon la revendication 4, dans lequel ledit dispositif à ultrasons intracorporel est adapté pour être inséré par le biais du vagin.

**13.** Système selon la revendication 4, dans lequel ledit dispositif à ultrasons intracorporel est adapté pour être inséré par le biais de l'urètre.

**14.** Système selon la revendication 4, dans lequel ledit dispositif à ultrasons intracorporel est adapté pour être inséré par le biais de l'oesophage.

**15.** Système selon la revendication 4, dans lequel ledit dispositif à ultrasons intracorporel est monté sur un mécanisme de transport.

**16.** Système selon la revendication 15, dans lequel ledit mécanisme de transport est sélectionné parmi le groupe comprenant une sonde endoscopique et un cathéter.

**17.** Système selon la revendication 2, dans lequel ledit système d'imagerie peut être actionné de façon à utiliser l'imagerie par impulsion-écho.

**18.** Système selon la revendication 2, dans lequel ledit système d'imagerie peut être actionné de façon à utiliser l'imagerie par diffusion inversée.

**19.** Système selon la revendication 2, dans lequel ledit système d'imagerie peut être actionné de façon à utiliser l'imagerie par résonance magnétique.

**20.** Système selon la revendication 2, dans lequel ledit système d'imagerie peut être actionné de façon à utiliser la tomographie informatisée thermo-acoustique.

**21.** Système selon les revendications 3 à 5, dans lequel ladite TUCT comprend une analyse de l'harmonie des fréquences.

**22.** Système selon les revendications 3 à 5, dans lequel ladite TUCT comprend une analyse des combinaisons des fréquences.

**23.** Système selon les revendications 3 à 5, dans lequel ladite TUCT comprend une analyse des combinaisons harmoniques des fréquences.

**24.** Système selon les revendications 3 à 5, dans lequel ladite TUCT est réalisée au moyen d'une analyse en spirale.

**25.** Système selon les revendications 3 à 5, dans lequel ladite TUCT comprend une analyse du temps de vol.

**26.** Système selon les revendications 3 à 5, dans lequel ladite TUCT comprend une analyse du déphasage.

**27.** Système selon les revendications 3 à 5, dans lequel ladite TUCT comprend une analyse de la dispersion de vitesse en fonction des fréquences.

**28.** Système selon la revendication 1 ou 2, étant non invasif.

**29.** Système selon la revendication 1 ou 4, étant le moins invasif possible.

```
        ┌─────────────────┐
        │       100       │
        │      begin      │
        └────────┬────────┘
                 │
 ┌───────────────┴─────────────────────────────────────┐
 │                     101                              │
 │ deliver bursts of the HIFU from different directions │
 │                and frequencies                       │
 └───────────────┬─────────────────────────────────────┘
                 │
 ┌───────────────┴─────────────────────────────────────┐
 │                     102                              │
 │ passively scan so as to receive ultrasonic radiation │
 │            with other frequencies                    │
 └───────────────┬─────────────────────────────────────┘
                 │
        ┌────────┴────────┐
        │       103       │
        │       end       │
        └─────────────────┘
```

Fig. 1

```
        ┌─────────────────────┐
        │        200          │
        │       begin         │
        └──────────┬──────────┘
                   │
        ┌──────────┴──────────────────────┐
        │              201                 │
        │  administer imaging contrast agent│
        └──────────┬───────────────────────┘
                   │
   ┌───────────────┴──────────────────────────┐
   │                202                        │
   │  image a region containing the target tissue│
   └───────────────┬──────────────────────────┘
                   │
        ┌──────────┴──────────┐
        │        203          │
        │ determine a focal region│
        └──────────┬──────────┘
                   │
   ┌───────────────┴──────────────────────────┐
   │                204                        │
   │ position the focal region onto the target tissue│
   └───────────────┬──────────────────────────┘
                   │
        ┌──────────┴──────────┐
        │        205          │
        │ damage the target tissue│
        └──────────┬──────────┘
                   │
        ┌──────────┴──────────┐
        │        206          │
        │ construct a temperature image│
        └──────────┬──────────┘
                   │
        ┌──────────┴──────────┐
        │        207          │
        │ determine damage extent│
        └──────────┬──────────┘
                   │
        ┌──────────┴──────────┐
        │        208          │
        │   image the region  │
        └──────────┬──────────┘
                   │
   ┌───────────────┴──────────────────────────┐
   │                209                        │
   │   assess damage to the target tissue      │
   └───────────────┬──────────────────────────┘
                   │
        ┌──────────┴──────────┐
        │        210          │
        │        end          │
        └─────────────────────┘
```

Fig. 2

Fig. 3

Fig. 4

```
                        ┌─────────────────┐
                        │      350        │
                        │     begin       │
                        └────────┬────────┘
                                 │
            ┌────────────────────┴────────────────────┐
            │                  351                     │
            │  insert an intracorporeal ultrasound device │
            └────────────────────┬────────────────────┘
                                 │
          ┌──────────────────────┴──────────────────────┐
          │                   352                        │
          │  position an extracorporeal ultrasound device │
          └──────────────────────┬──────────────────────┘
                                 │
              ┌──────────────────┴──────────────────┐
              │                353                   │
              │     transmit ultrasonic radiation    │
              └──────────────────┬──────────────────┘
                                 │
                 ┌───────────────┴───────────────┐
                 │             355               │
                 │        scan the region        │
                 └───────────────┬───────────────┘
                                 │
              ┌──────────────────┴──────────────────┐
              │                354                   │
              │    analyze the ultrasonic radiation   │
              └──────────────────┬──────────────────┘
                                 │
                        ┌─────────────────┐
                        │      356        │
                        │      end        │
                        └─────────────────┘
```

# Fig. 5

Fig. 6A

Fig. 6B

Fig. 6C

```
        ┌─────────────────────┐
        │        400          │
        │       begin         │
        └─────────────────────┘
                   │
    ┌──────────────────────────────────────────┐
    │                  401                       │
    │  image a region containing the target tissue │
    └──────────────────────────────────────────┘
                   │
        ┌─────────────────────────┐
        │          402            │
        │   damage the target tissue │
        └─────────────────────────┘
                   │
        ┌─────────────────────────┐
        │          403            │
        │  construct a temperature image │
        └─────────────────────────┘
                   │
        ┌─────────────────────────┐
        │          404            │
        │   determine damage extent │
        └─────────────────────────┘
                   │
        ┌─────────────────────┐
        │        405          │
        │        end          │
        └─────────────────────┘
```

# Fig. 7

Fig. 8

Fig. 9A

Fig. 9B

Fig. 9C

Fig. 10A

**34A**

**26A**

High Intensity
Focused
Ultrasonic Waves

| Signal Generator | Power Amplifier |

**86**

**88**

Main Computer

HIFU
ARRAY

**22**

**20A**

**82**

X-Motor

**83**

Motion Control

Y-Motor

**85**

**80**

Z-Motor

**87**

Encoders

**84**

Fig. 10B

Fig. 10C

Fig. 11

151    150    Fig. 12A

Fig. 12B

Fig. 12C

Fig. 13A

Fig. 13B

Fig. 14

Fig. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6500121 B
- US 20040030227 A
- US 4509368 A
- US 6636584 B
- US 6587540 B
- US 6005916 A
- US 5588032 A
- US 6216025 B
- US 20050038339 A
- US 6716184 B
- US 6685639 B
- US 6280402 B
- US 5769790 A
- US 5558092 A
- US 4932414 A
- WO 9940847 A
- US 20030105402 A
- US 6916290 B
- US 4385634 A

### Non-patent literature cited in the description

- **RANDAL J.** High Intensity Focused Ultrasound Makes Its Debut. *Journal of the National Cancer Institute,* 03 July 2002, vol. 94 (13), 962-864
- **HYNYNEN K ; POMEROY O ; SMITH D ; HUBER P ; MCDANNOLD N ; KETTENBACH J ; BAUM J ; SINGER S ; JOLESZ F. MR.** Imaging-Guided Focused Ultrasound Surgery Of Fibroadenomas. *The Breast: A Feasibility Study, Radiology,* 21 April 2001, vol. 9, 176-185
- **GIANFELICE D ; KHIAT A ; AMARA M ; BELBLIDIA A ; BOULANGER Y. MR.** Imaging-Guided Focused Ultrasound Ablation Of Breast Cancer: Histopathologic Assessment Of Efficacy - Initial Experience. *Radiology,* 2003, vol. 227 (3), 849-855
- **HUBER PE ; JENNE JW ; RASTERT R ; SIMIAN-TONAKIS I ; SINN HP et al.** A New Noninvasive Approach In Breast Cancer Therapy Using Magnetic Resonance Imaging-Guided Focused Ultrasound Surgery. *Cancer Res,* 01 December 2001, vol. 61 (23), 8441-7
- **GIANFELICE D ; ABDESSLEM K ; BOULANGER Y ; AMARA M ; BELBLIDIA A.** MR Imaging-Guided Focused Ultrasound Surgery (Mrigfus) Of Breast Cancer: Correlation Between Dynamic Contrast-Enhanced MRI And Histopathologic Findings. *RSNA,* 2002
- **TEMPANY CMC ; STEWART EA ; MCDANNOLD N ; QUADE B ; JOLESZ F ; HYNYNEN K.** MRI Guided Focused Ultrasound Surgery (FUS) Of Uterine Leiomyomas: A Feasibility Study. *Radiology,* 2003, vol. 227, 897-905
- **WU F ; CHEN WZ ; BAI J ; ZOU JZ ; WANG ZL ; ZHU H ; WANG ZB.** Tumor Vessel Destruction Resulting From High-Intensity Focused Ultrasound In Patients With Solid Malignancies. *Ultrasound Med Biol,* April 2002, vol. 28 (4), 535-542
- **MADERSBACHER S ; SCHATZL G ; DJAVAN B ; STULNIG T ; MARBERGER M.** Long-Term Outcome Of Transrectal High-Intensity Focused Ultrasound Therapy For Benign Prostatic Hyperplasia. *Eur. Urology.,* 2000, vol. 37, 687-694
- **S. A. JOHNSON ; D. A. CHRISTANSEN ; C. C. JOHNSON ; J. F. GREENLEAF ; B. RAJAGOPALAN.** Non-Intrusive Measurement of Microwave and Ultrasound-Induced Hyperthermia by Acoustic Temperature Tomography. *Ultrasonics Symposium Proceedings,* 1977, 977-982
- **HYNYNEN K ; POMEROY O ; SMITH D ; HUBER P ; MCDANNOLD N ; KETTENBACH J ; BAUM J ; SINGER S ; JOLESZ F.** MR Imaging-Guided Focused Ultrasound Surgery Of Fibroadenomas. *The Breast: A Feasibility Study, Radiology,* April 2001, vol. 21 (9), 176-185
- **GIANFELICE D ; KHIAT A ; AMARA M ; BELBLIDIA A ; BOULANGER Y.** MR Imaging-Guided Focused Ultrasound Ablation Of Breast Cancer: Histopathologic Assessment Of Efficacy - Initial Experience. *Radiology,* 2003, vol. 227 (3), 849-855
- Variation of acoustic speed with temperature in various excised human tissues studied by ultrasound computerized tomography. **RAJAGOPALAN, B. ; GREENLEAF, J. F. ; THOMAS, P. J. ; JOHNSON, S. A. ; BAHN, R. C.** Ultrasonic Tissue Characterisation II. 1979, 227-33

- **JOSSINET, J. ; CATHIGNOL, D. ; CHAPELON, J. Y. ; DITTMAR, A. ; SCHMITT, M.** Practical temperature measurements by ultrasound tomography. *Journal de Biophysique & Medicine Nucleaire,* 1983, vol. 7 (5), 179-83
- **MIZUTANI, K. ; NISHIZAKI, K. ; NAGAI, K. ; HARAKAWA, K.** Measurement of temperature distribution in space using ultrasound computerized tomography. *Japanese Journal of Applied Physics,* May 1997, vol. 36 (5B), 3176-7
- **AZHARI H ; SAZBON D.** Volumetric imaging using spiral ultrasonic computed tomography. *Radiology,* 1999, vol. 212 (1), 270-275
- **MOSTAFA FATEMI ; LESTER E. WOLD ; AZRA ALIZAD ; JAMES F. GREENLEAF.** *Vibro-Acoustic Tissue Mammography, IEEE Transactions On Medical Imaging,* 2001, vol. 21, 1
- **AZHARI H. ; SAZBON D.** Volumetric imaging using spiral ultrasonic computed tomography. *Radiology,* 1999, vol. 212 (1), 270-275
- **GREENLEAF JAMES F. ; BAHN, ROBERT C.** CLINICAL IMAGING WITH TRANSMISSIVE ULTRASONIC COMPUTERIZED TOMOGRAPHY. *IEEE Trans, Biomed Eng,* February 1981, vol. BME-28 (2), 177-185
- **JAGO, J. R. ; WHITTINGHAM, T. A.** Practical system for the application of ultrasound computed tomography to medical imaging. *IEE CONF PUBL, the International Conference on Acoustic Sensing and Imaging CONFERENCE LOCATION- London,* 29 March 1993, 257-265